# EUROPEAN PATENT SPECIFICATION

(11) **EP 4 572 721 B1**
(45) Date of publication and mention of the grant of the patent: **17.09.2025**
(21) Application number: 24735933.4
(22) Date of filing: 19.06.2024
(51) Int. Cl.: A61F 13/15, A61F 13/42

(54) **METHOD OF JOINING AN ABSORBENT ARTICLE AND A CONTINUOUS STRIP**
VERFAHREN ZUM VERBINDEN EINES SAUGFÄHIGEN ARTIKELS UND EINES KONTINUIERLICHEN STREIFENS
PROCÉDÉ D'ASSEMBLAGE D'UN ARTICLE ABSORBANT ET D'UNE BANDE CONTINUE

(30) Priority: 19.06.2023 DE 102023116003
(43) Date of publication of application: 25.06.2025
(73) Proprietor: AssistMe GmbH, 10555 Berlin (DE)
(72) Inventor: BRANDL, Julio, 10555 Berlin (DE); ANIELSKI, Alexander, 10555 Berlin (DE); KATHAN, Jonas, 10555 Berlin (DE); HARRIS, Tomos, 10555 Berlin (DE)
(74) Representative: Gulde & Partner
(86) International application number: PCT/EP2024/067111
(87) International publication number: WO 2024/261066

(56) References cited:
- EP-A1- 3 936 099
- US-A1- 2022 354 708
- US-B1- 6 200 250

## Description

The invention relates to a method of joining an absorbent article and a continuous strip comprising a moisture sensor strip and a cover layer. Further it relates to a continuous reel of a continuous strip for use in the method according to the invention, an apparatus for use in method according to the invention and a system manufactured in the method according to the invention.

One of the biggest challenges that modern societies have to face in coming years is the aging of their populations. The number of caregivers and elderly at nursing homes will become less balanced, therefore less caregivers will attend more residents, which raises several difficulties. Already today caregivers do check on residents according to a tight time schedule, not only making it an exhausting workload but also rendering it difficult to figure out, which urgent need to attend first. Also, important to note is that caregivers have assigned a certain number of residents they have to monitor on each shift, which renders efficient scheduling even more important.

In home care situations, family caregivers are also confronted with similar problems and have to take care of a cared-for person in addition to their other duties such as work, other family members and other obligations, so that they are under considerable time pressure and are exposed to high levels of stress. Baby care is also a time-consuming task for private households and parents or caregivers.

There are, among others, two circumstances that cause the need for absorbent articles. Firstly, the circumstance of incontinence, which can be understood as a disease that can have many different causes, and the circumstance in the field of infant care, which is related to the lack of independent control of the child over his excretions.

Incontinence is a condition in which there is uncontrolled release of natural discharges or evacuations from the bladder and/or bowel. Urinary incontinence refers to loss of bladder control resulting in involuntary or uncontrolled urination. While some forms of incontinence, particularly urinary/bladder incontinence are relatively widespread, the condition typically affects the elderly and the infirm and is more prevalent among women.

Another current pain point at care homes are night shifts, wherein one of the main challenges is the wide area of the care home, and the reduced numbers of caregivers during these shifts. Therefore, the physical strain on caregivers becomes a problem.

Further, due to the lack of caregivers, it often occurs that caregivers have to fill in for others or are hired on a temporary basis from external sources. In such cases, it is important to ensure rapid familiarization and thus smooth integration into the nursing home's workflow.

US 2022 / 0 401 269 A1 and WO 2021/ 064 121 A1 describe a method for bonding an absorbent article to a moisture monitoring system comprising a top layer and a sensor film with a sensor system printed on a sheet. Furthermore, a gap created by the sensor film, an adhesive and the backsheet of the absorbent article as well as a pocket for a measuring unit are described.

EP 3 451 988 B1 and WO 2018/ 229 017 A1 describe a substrate for an absorbent article having a slit, comprising sensor tracks, a strip layer optionally bonded to a side of the substrate facing the skin to form a pocket, an insulating layer bonded to a first surface of the substrate on the sensor tracks, and a channel-shaped pocket formed between the first surface and the insulating layer.

WO 2018/ 229 280 A1 discloses the substrate from EP 3 451 988 B1 and describes system and electrotechnical aspects of a measuring system.

EP 4 014 936 A1 discloses the substrate from EP 3 451 988 B1 and describes a sensor system for measuring several physical properties.

WO 2021/ 224 033 A1 discloses the substrate from EP 3 451 988 B1 and describes a structure of a detection device.

US 2020/0 256 819 A1 describes a detection sensor comprising an optional peelable sensor substrate for attaching the detection sensor to clothing, an optional adhesive layer, a sensor substrate, a conductive layer produced on the upper surface of the sensor substrate with two sensor electrodes with two sensor terminals, a feces-sensitive layer, an adhesive layer - optionally for attaching the detection sensor to the clothing or for attaching an optional non-woven layer.

WO 2022/ 008 584 A1 and EP 3 936 099 A1 describe a manufacturing method for an absorbent article comprising providing a backsheet printed with a conductive pattern, a cover strip layer and an absorbent core layer, cutting an opening in the backsheet, and sealing the cover strip layer located between the backsheet and the absorbent core layer to the backsheet by means of an adhesive pattern on the cover strip layer or on the backsheet forming a pocket between the edges and the front and/or rear end of the cover strip layer.

It is thus an object of the present disclosure to overcome or reduce at least some of the drawbacks of the prior art and to provide a method of manufacturing absorbent articles comprising a sensor that provides automatic detection of moisture in the absorbent article. In this regard, the article is to be characterized by ease of handling.

The given task is solved by the subject-matter of the independent claims. Advantageous embodiments of the invention can further be gained from the dependent claims.

A first aspect of the disclosure refers to a method of joining an absorbent article and a continuous strip comprising a moisture sensor strip and a cover layer. The method (basic embodiment) comprises a step of adhering a portion of the moisture sensor strip to a first partial surface of the absorbent article by applying a first adhesive agent between the moisture sensor strip and the first partial surface, wherein in a pocket region of the absorbent article there is no adhesion of the moisture sensor strip to the first partial surface. A further step of the method comprises adhering a portion of the cover layer to a second partial surface of the absorbent article by applying a second adhesive agent between the cover layer and the second partial surface, wherein in the pocket region of the absorbent article lateral edge regions of the cover layer are adhered to the absorbent article, while a region between the lateral edge regions remains adhesive-free and wherein the cover layer overlaps both longitudinal edges of the moisture sensor strip. In a further step the adhered portion of the moisture sensor strip and the adhered portion of the cover layer are separated from the continuous strip. Furthermore, an access opening to a pocket space confined by the pocket region of the absorbent article, by the lateral edge regions of the cover layer and by the region between the lateral edge regions, is provided, wherein the pocket space is accessible via the access opening. The pocket space is configured for receiving a clip comprising an integrated circuit, a first pivotable element and a second pivotable element connected to the first pivotable element at a folding edge, wherein a size of the pocket space is fitted to the size of the first pivotable element, being pierced by teeth of the second pivotable element in a closed position of the clip for attaching the clip to the cover layer, and aligning the clip with the moisture sensor strip such that contact pads of the moisture sensor strip and contact pins of the clip are conductively connected to each other and such that the moisture sensor strip is aligned between the teeth.

Herein, a method is provided for converting an absorbent article into an absorbent article comprising a sensor suitable for measuring a moisture, which may be done either during the production process of the absorbent article or after the absorbent article has been manufactured.

Dielectric spectroscopy is preferably used as a suitable measurement method. Here, an observation of magnitude and phase shift between applied sinusoidal voltage and measured current is performed. This method allows the separation of conductive (real) and capacitive (imaginary) parts of the impedance, furthermore a 4-wire measurement increases the accuracy of the impedance measurements. The product manufactured in the process according to the invention is thus designed to measure moisture using said method. Accordingly, advantages mentioned in the further course, which influence properties within a measuring method by the design, are always directed to the mentioned measuring method.

Absorbent articles include, but are not limited to, hygiene and sanitary products, including for example (disposable) diapers, sanitary napkins, panty liners, incontinence pads and sweat wipes. These can find application in humans but also in animals, such as pets. The absorbent article absorbs liquid released by the wearer and includes, for example, liquid excreta and body fluids. Absorbent articles represent a particularly broad field of application as incontinence products, especially in the form of diapers for adults and children. Absorbent articles include, in particular, template products that are converted into a final product in further process steps.

The continuous strip comprises a strip that can be characterized by the fact that it can be quasi-endless. Obviously, it is not mathematically infinite. For practical purposes, quasi-endless means that it can be used several times in the present process. In other words, it is capable of being run through the procedure several times. It comprises repeating components, so that for each run of the process, the elements required for the process are provided by the continuous strip, without the continuous strip being consumed after application of the process. Instead, it can be used again for the repetition of the process to produce the process product.

The moisture sensor strip comprises a strip suitable for measuring humidity. For the present method, this is to be understood as meaning that the sensor comprises at least the components that detect the measurement signal for the first time. This can be generally referred to as a measuring sensor, whereby no sensor is meant in the literal sense. In other words, it is to be understood here as the measuring means that detects the desired measurand by means of the underlying measuring method. In addition, the sensor may also include other components, such as those for processing the measurement signal.

The cover layer comprises a strip forming an external layer and which acts as a shielding layer, further minimizing interference from outside sources during the sensing process, and further providing enhanced comfort for the wearer. It also protects the moisture sensor strip by covering it. The cover layer should preferably be breathable, for reasons of comfort, tin particular, the cover layer should not have any hydrophilic properties so as to have little effect on the measurement process. In particular, the measurement is then not distorted during a capacitance measurement. In addition, the cover layer preferably has a thickness that is designed in such a way that it shields the sensor arranged underneath it. This means that external influences, such as pressing against the inside of the thigh, do not lead to an increase in capacitance. An appropriately designed thickness also ensures sufficient tensile strength, which simplifies processing in production lines.

The term strip, which is used for the continuous strip and for the cover layer, is by no means bound to a specific shape. Thus, various shapes can be considered. For example, the shape of the humidity sensor strip can be rectangular, square, circular, elliptical and more. The same goes for the cover layer.

The first adhesive agent and the second adhesive agent comprise such types and kinds of adhesives suitable for bonding the respective elements according to the present method according to the invention. Accordingly, the first adhesive and the second adhesive may differ from each other. In addition, however, the first adhesive and the second adhesive may comprise the same adhesive.

The use of the first adhesive agent fixes the moisture sensor strip to its place of application and also ensures a uniform distance between the sensor surface and the absorbent article. Depending on the measurement method used, this increases measurement accuracy by preventing movement or user positioning, etc. In particular, it reduces a capacitance deviation of different absorbent articles joint with the continuous strip. The first adhesive agent and the second adhesive agent also provide increased insulation from liquid. The first adhesive agent and the second adhesive agent should each be compatible with the surfaces to which they are applied. Preferably, they have low surface energies.

The thickness of the applied first adhesive agent is selected such that the moisture sensor strip is insulated from and reliably bonded to the absorbent article. In addition, the thickness is selected in such a way that the penetration depth of the moisture sensor strip is reduced, given the appropriate measuring method. The adhesive is preferably homogeneous in order to reduce variations in the recorded readings of different absorbent articles or non-uniform effects due to swelling of a core of the absorbent article, which lead to a high variance in the recorded readings.

The first partial surface and the second partial surface of the absorbent article comprise different shapes and preferably have shapes corresponding to the portion of the moisture sensor strip and the portion of the cover layer, respectively. In particular, the second partial surface may comprise the first partial surface.

In the area of the pocket space, there is no first adhesive agent between the moisture sensor strip and the first partial surface of the absorbent article, so that a contact element for a conductive connection of further hardware components can be connected through the pocket space to electrodes of the moisture sensor strip in a removable manner.

Using the method, any absorbent article can be easily converted into an absorbent article having a measuring device or an absorbent article suitable for measuring moisture, without having to modify the main materials or components of the absorbent article. A production line for absorbent articles can be modified so that the method according to the invention can be integrated into the process for producing the absorbent article. Alternatively, the process according to the invention can be applied to an existing absorbent article after its manufacturing process.

According to the present disclosure, the pocket space is configured for receiving a clip comprising an integrated circuit, a first pivotable element and a second pivotable element connected to the first pivotable element at a folding edge, wherein a size of the pocket space is fitted to the size of the first pivotable element. Furthermore, the pocket space is configured for being pierced by teeth of the second pivotable element in a closed position of the clip for attaching the clip to the cover layer. Moreover, the pocket space is configured for aligning the clip with the moisture sensor strip such that contact pads of the moisture sensor strip and contact pins of the clip are conductively connected to each other and such that the moisture sensor strip is aligned between the teeth. In other words, a suitable clip has two pivotable parts. The first pivotable element is inserted into the pocket space, and the second pivotable element is used to attach to the pocket space and provide contact between the clip and the sensor. The teeth of the clip pierce the cover layer to hold it in place. Because of the pocket space formed by the method of the invention, the pocket space is configured to be tight enough to align the clip with the moisture sensor strip. At the same time, the dimensions of the pocket space are such that the contact pins can be correctly and easily aligned with the contact pads such as circumferential traces of interdigitated electrodes, while at the same time holding the clip securely in place. Accordingly, the material of the cover layer includes a material selected with a sufficiently low grammage that allows the teeth to pierce the pocket space to ensure that the clip remains in place. The clip clamps the moisture sensor strip between the contact pins of the clip and the flexible parts of the closure hinge. This puts pressure on the back of the moisture sensor strip and pushes it against the contact pins.

Preferably, in the method are a plurality of identical pocket portions with a plurality of corresponding lateral edge regions of the cover layer and a plurality of regions between the lateral edge regions formed, such that a corresponding plurality of pocket spaces confined by them are formed as well. Thus, it is possible to have pocket spaces at different places, therefore having the possibility to attach a clip comprising hardware on a place by choice.

In a first preferred embodiment (so called roll-to-roll process) of the present disclosure, the continuous strip is provided as a continuous reel comprising the moisture sensor strip adhered to the cover layer and wherein a portion of the continuous strip comprising the portion of the moisture sensor strip and the portion of the cover layer is unrolled before being adhered to the absorbent article. Accordingly, the moisture sensor strip and the cover layer are not used separately from one another in the present method, but as a kind of semi-finished product, i.e. prefabricated raw material, or also as a kind of laminate comprising the two components mentioned. The moisture sensor strip and the cover layer are also bonded to one another, in accordance with the relative arrangement to one another according to the invention. Hereby, the application of both components is considerably simplified and at the same time made more precise. Because they are already bonded together, the two components can no longer slip relative to each other. Thus, it is not necessary to simultaneously ensure a correct arrangement of the moisture sensor strip relative to the absorbent article, of the cover layer relative to the absorbent article and of the moisture sensor strip relative to the cover layer, but only a correct arrangement of the glued-together component comprising the portion of the moisture sensor strip and the portion of the cover layer relative to the absorbent article. Moreover, due to the unrolling, this control is carried out continuously and section by section. Even after application of the portion of the moisture sensor strip and the portion of the cover layer to the absorbent article, both components can no longer slip or shift, but remain in their arrangement.

According to a second preferred embodiment (also called roll-to-roll process) of the invention, the moisture sensor strip is provided as a first continuous reel and the cover layer is provided as a second continuous reel. This provides flexibility during the process. By providing the moisture sensor strip and the cover layer in the form of a first continuous reel and a second continuous reel, it is possible that the cover layer, after application of the moisture sensor strip from the first continuous reel to the absorbent article, covers not only the longitudinal edges of the moisture sensor strip, but also the transversal edges. As a result, the moisture sensor strip is completely covered by the cover layer and is overlapped on all its sides, which provides it with additional protection. In addition, the provision of two reels enables the exchange of the reels. Depending on the application, there may be different requirements for the moisture sensor strip or the cover layer. By providing both separately, a suitable moisture sensor strip or cover layer can be selected depending on the application, which proves to be particularly suitable for the absorbent article. This also ensures that the cover layer is adapted to the moisture sensor strip.

A first variant of the second preferred embodiment of the method before adhering the portion of the moisture sensor strip to the first partial surface of the absorbent article and before adhering the portion of the cover layer to the second partial surface of the absorbent article, comprises the step of adhering the moisture sensor strip to the cover layer by applying a third adhesive agent between at least a part of the moisture sensor strip and a part of the cover layer. This results in a combination of the advantage of increased precision in the arrangement of the moisture sensor strip and the cover layer relative to each other and relative to the absorbent article, and the advantage of increased flexibility, since the moisture sensor strip and cover layer used are advantageously matched to each other.

According to a second variant of the second preferred embodiment of the invention, after adhering the portion of the moisture sensor strip to the first partial surface of the absorbent article, the method further comprises the step of adhering the moisture sensor strip to the cover layer by applying a third adhesive agent between the moisture sensor strip and the cover layer. In this case, the moisture sensor strip is placed on the absorbent article and already fixed by the fabric closure before the cover layer is adhered to the moisture sensor strip and the absorbent article. In this way, the user of the method can always select and control whether and how far the cover layer overlaps the moisture sensor strip at the transversal edges, while at the same time ensuring that the moisture sensor strip is fixed and does not slip when the cover layer is applied.

In a further preferred embodiment of the second variant of the second preferred embodiment, the moisture sensor strip remains adhesive-free in the pocket region of the absorbent article in the region between the lateral edge regions. Accordingly, the moisture sensor strip is freely movable in the pocket region. This prevents an adhered moisture sensor strip in the pocket region from not being ideally connectable to any hardware that may be electrically connected to the moisture sensor strip. By remaining freely movable, it can be aligned as desired and any inaccuracies due to tolerances can be compensated for.

In a further preferred embodiment of the first preferred embodiment or the first variant of the second preferred embodiment, the moisture sensor strip is intermittently adhered to the cover layer, wherein a non-adhered region of the moisture sensor strip is overlaid with the pocket region before adhering the moisture sensor strip to the absorbent article. This combines the advantage of having the moisture sensor strip and cover layer adhered together prior to adhering to the absorbent article with the advantage of having the moisture sensor strip remain adhesive-free in a pocket area and thus movable.

According to an third preferred embodiment (so called retrofitting), the continuous reel further comprises the first adhesive agent, the second adhesive agent and at least one release paper for covering the first adhesive agent and the second adhesive agent and wherein the method further comprises the step of removing the release paper after unrolling the portion of the continuous strip and before adhering the portion of the moisture sensor strip and/or before adhering the portion of the cover layer to the absorbent article. This further simplifies the application of the portion of the moisture sensor strip and the portion of the cover layer to the absorbent article. The first adhesive agent and the second adhesive agent are already present for adhering and do not need to be additionally applied. It also avoids the need to apply an adhesive to the absorbent article. This requires a high degree of precision to prevent the presence of excess adhesive that would either have to be removed or would adversely affect the backing. In addition, the adhesive is exposed in a simple manner by removing the release paper. In addition, complicated additional equipment for applying the adhesive agents can be avoided, which enables a larger group of people especially in healthcare facilities to adhere the portion of the moisture sensor strip and the portion of the cover layer to the absorbent article.

A preferred embodiment of the third preferred embodiment further comprises the step of winding the removed release paper onto a release paper roll and applying pressure to the portion of the continuous strip with a pressure roller while adhering the portion of the moisture sensor strip and the portion of the cover layer to the absorbent article, wherein the continuous reel and the release paper roll are connected to each other by a transmission comprising a coupling. The connection of the continuous reel and the release paper roll exerts a permanent tension on the portion of the continuous strip so that the unrolling process of the portion of the continuous strip is not interrupted. By applying pressure to the portion of the continuous strip with the pressure roller while adhering the portion of the moisture sensor strip and the portion of the cover layer to the absorbent article, a reliable material bond of the portion of the moisture sensor strip and the portion of the cover layer to the absorbent article is established. In addition, the unwound release paper is automatically rolled up so that it does not interfere with the user.

In the third preferred embodiment, the pressure of the pressure roller is preferably adjusted by a spring preload. This allows a static pressure provided by the pressure roller to be varied and adjusted at any time. This is particularly necessary for absorbent articles with different thicknesses. In these cases, it is advantageous to adjust the pressure applied in order to always ensure a contact pressure necessary for reliable bonding.

In a preferred method according to the third preferred embodiment the pressure is applied by a first pressure roller and a second pressure roller forming a gap into which the absorbent article is inserted for adhering the portion of the moisture sensor strip and the portion of the cover layer to the absorbent article. This additionally reduces incorrect application. The first pressure roller and the second pressure roller and the gap formed between them provide a guide which, on the one hand, exerts a suitable contact pressure and, on the other hand, forms a guide which makes it easier for the user to correctly connect the portion of the moisture sensor strip and the portion of the cover layer to the absorbent article.

In a preferred embodiment of the third preferred embodiment, an end of the portion of the continuous strip is disposed in front of the first pressure roller and the second pressure roller and the absorbent article receives the end when the portion of the continuous strip is inserted in the gap. This makes it even easier for the user to apply the process. The release paper is already unrolled so that the absorbent article comes into contact with the exposed first adhesive and/or second adhesive. As a result, the portion of the continuous strip adheres to the absorbent article and progressively arranges itself on the absorbent article when the absorbent article is inserted. In other words, the user only need to insert the absorbent article into the gap. The arrangement of the portion of the continuous strip on the absorbent article and the adhesion to each other is automatic. Removing the release paper from the portion of the continuous strip and rolling it up is also automatic.

Another aspect of the present disclosure relates to a continuous reel (basic embodiment) of a continuous strip for use in any one of the method according to the basic embodiment, the first preferred embodiment and the third preferred embodiment of the invention or a preferred embodiment of the foregoing, wherein the continuous reel comprises a moisture sensor strip comprising two interdigitated electrodes (IDE) printed with conductive materials-based ink on a polymer substrate, prefereably polyethylenterephthalat, PET, substrate, a cover layer comprising a non-woven material and an access opening to a pocket space and a third adhesive agent at least intermittently disposed between a first surface of the moisture sensor strip and a first surface of the cover layer material, wherein no third adhesive agent is disposed in the pocket space. The continuous reel further comprises a plurality of identical segments in a lengthwise direction of the continuous reel, each segment comprising a first adhesive agent disposed on a second surface of the moisture sensor strip except for a pocket portion of the moisture sensor strip, a second adhesive agent disposed on a first surface of the cover layer, wherein in a pocket portion of the cover layer the second adhesive is disposed on lateral edge regions of the pocket portion of the cover layer and a region between the lateral edge regions is free of the second adhesive agent, and comprising at least one release paper covering the first adhesive agent and the second adhesive agent. After a portion of the continuous strip is joint with an absorbent article and optionally connected to a sensor clip comprising the further hardware, for example an integrated circuit for the above-mentioned dielectric spectroscopy, a moisture/fill level of the absorbent article can preferably be determined based on a capacitance determination via the IDE, where the capacitance depends on a wetting of an absorbent core of the absorbent article and an associated change in its dielectric permittivity. The IDE provides a suitable field penetration depth for measuring the dielectric properties of the absorbent core, but not a body of a wearer. The penetration depth is the point at which an electromagnetic wave only corresponds to 1/e (approx. 37%) of the initial amplitude when penetrating a medium. Both interdigitated conductive areas are spaced and electrically insulated to each other. The interdigital structure of the sensor generates a fringe field with a limited range, the penetration depth, which interacts with dielectric materials within the penetration depth. The interdigital electrode is printed on the surface of the PET substrate, which, when in contact with a proper clip, allows impedance measurements to be made along the entire length of the moisture sensor strip and the capacitance values for the absorbent article to be determined, enabling the degree of filling of the absorbent article to be determined using a suitable calculation algorithm. Furthermore, the portion of the moisture sensor strip is coupled to the absorbance material only capacitively, without electrical contact. A wetting of the absorbent core with a water-based solution changes the dielectric permittivity of the absorbent core, that leads to a capacitance change, that is measurable. Due to the interdigital structure, the sensor has a maximum penetration depth of about 10 millimetres, which is sufficient to measure the dielectric properties of the absorbing core, but not the body of the absorbent article carrier. The moisture sensor strip comprises a flexible and flat PET substrate that allows the product to attach to the outside of an absorbent article and conform to its shape during manufacturing, folding, packaging, unpacking and wearing by the user/patient. Preferably, the IDE is produced using roll-2-roll screen printing, while other methods, such as flexographic or (roto)gravure may also be used.

The conductive materials-based ink comprises preferably printable conductive materials in particular conductive carbon materials (particles) in a polymer binder, conducting polymers, copper and/or silver. In particular, conductive carbon materials (particles) in a polymer binder make the IDE flexible and allow measurements to be taken while the user/patient is wearing the absorbent article, regardless of their position of movement. Carbon is an abundant resource and inexpensive. The particles are held together in the printing paste by a nonconductive binder. There is no sintering of the particles. The cost is competitive with metal pastes and the particles have a smaller environmental footprint.

The ink imprint can be produced using additive manufacturing processes such as rotary screen printing, flexographic or gravure printing. These processes are highly repeatable, fast and cost-effective, and can be used in a roll to roll manner. The carbon-based ink has high scratch resistance and low electrical contact resistance, so contact pins of a clip can be easily connected to the sensor. The carbon layer is oxidation resistant under standard conditions, meaning no top layer is required as with etched copper-clad laminates (flexPCBs) or printed copper or silver tracks, and the contact points do not need to be plated (e.g., with gold). The cured carbon-based ink is stable when bent at the bending diameters expected in absorbent article production, so that no significant reduction in conductivity occurs, which was measured down to a bending diameter of 8 mm.

PET is inexpensive, flexible and non-stretchable. It can be cured at temperatures up to 130° C, which allows fast curing of the conductive ink. PET can be heat treated to prevent substrate shrinkage while the ink is curing, and the surface can be modified to improve printability and paste adhesion. PET also has high chemical resistance, such as to organic solvents often used in conductive screen-printing pastes. Printing on a flat substrate such as a PET film also has the advantage of better conductivity, since ink does not penetrate the porous structure, and better structural stability, since bending porous substrates can deform the impregnating conductive tracks.

Accordingly, the moisture sensor strip and the cover layer are not used separately from one another in the method according to the basic embodiment, the first preferred embodiment and the third preferred embodiment of the invention or a preferred embodiment of the foregoing, but as a kind of semi-finished product, i.e. prefabricated raw material, or also as a kind of laminate comprising the two components mentioned. The moisture sensor strip and the cover layer are also bonded to one another, in accordance with the relative arrangement to one another according to the invention. Hereby, the application of both components is considerably simplified and at the same time made more precise. Because they are already bonded together, the two components can no longer slip relative to each other. Thus, it is not necessary to simultaneously ensure a correct arrangement of the moisture sensor strip relative to the absorbent article, of the cover layer relative to the absorbent article and of the moisture sensor strip relative to the cover layer, but only a correct arrangement of the glued-together component comprising the portion of the moisture sensor strip and the portion of the cover layer relative to the absorbent article.

The third adhesive may be the same as the first adhesive and/or the second adhesive, but may also be different from both.

According to a further preferred embodiment of the basic embodiment, the continuous reel comprises predefined separation areas for separating segments of the continuous reel, the separation areas being provided on the moisture sensor strip and the cover layer. Preferably, these areas include a perforation. On the one hand, this indicates to the user a suitable location for cutting off segments of the continuous reel. In addition, the severing process is made considerably easier. The user does not need any additional tools such as scissors or a knife. Handling is also simplified and, in particular, made more precise, since inaccuracies can be eliminated.

According to the disclosure, the continuous reel comprises a plurality of identical segments in a lengthwise direction of the continuous reel, each segment comprising a first adhesive agent disposed on a second surface of the moisture sensor strip except for a pocket portion of the moisture sensor strip and a second adhesive agent disposed on a first surface of the cover layer, wherein in a pocket portion of the cover layer the second adhesive is disposed on lateral edge regions of the pocket portion of the cover layer and a region between the lateral edge regions is free of the second adhesive agent. The continuous reel further comprises at least one release paper covering the first adhesive agent and the second adhesive agent. This provides the user with a continuous reel for use in the process according to the invention, whereby the moisture sensor strip is adhered to the cover layer, thereby considerably simplifying the application of the two components and at the same time making it more precise. All necessary adhesive agents are provided by the continuous reel, the arrangement of moisture sensor strip and cover layer is fixed and in connection with the process according to the invention the advantages described above are achieved.

According to an additionally preferred embodiment, the separation areas are disposed between the identical segments. This provides a continuous reel that provides a plurality of identical segments that are suitable for attachment to an absorbent article and can be easily separated from each other. The user is thus provided with a product that enables him to subsequently provide a variety of absorbent articles with a portion of the continuous strip in a simple and practical manner.

Another aspect of the present disclosure relates to a first continuous reel for use in the method according to the second preferred embodiment of the invention or a variant thereof and for manufacturing the continuous reel according to its basic embodiment, preferred embodiments of the basic embodiment and first embodiment of the continuous reel according to the invention, comprising a moisture sensor strip comprising two interdigitated electrodes printed with conductive materials-based ink on a polymer substrate, preferably polyethylenterephthalat, PET, substrate, wherein a distance between outer edges of the two interdigitated electrodes is 10 mm to 25 mm, preferably 13 mm to 22 mm, further preferably 15 mm to 18 mm, particularly preferably 16 mm, and preferably comprising a plurality of pairs of two interdigitated electrodes, each printed with conductive materials-based ink on the PET substrate, arranged laterally next to each other. This provides the first continuous reel for the method according to the invention, which in particular can be manufactured simply and inexpensively. The side-by-side arrangement in particular offers efficient manufacturing processes such as roll-to-roll manufacturing processes. The roll-to-roll production of the moisture sensor strip on PET allows low cost and highly conductive sensors to be produced, which allows impedance spectroscopy to be used. Herein a method is provided for producing a low-cost standalone moisture sensor strip roll based on printed electronics. In other words, the moisture sensor strip preferably has a width corresponding to the above-mentioned values of the distance between the outer edges of the electrodes. A corresponding distance between the outer edges of the two interdigitated electrodes has proven to be particularly advantageous. The area of the sensor has been reduced to such an extent that measurement noise is manageable and the signal remains reliable enough for an evaluation algorithm to work with. At the same time, the costs for the first continuous reel can be reduced while taking the aforementioned criteria into account. In particular for use in the method according to the second preferred embodiment of the invention, manufacturing the continuous reel (basic embodiment, preferred embodiment of the basic embodiment and first embodiment of the continuous reel according to the invention) and for retrofitting with this manufactured continuous reel, this provides an advantageous first continuous reel, the advantages of which are further improved by the following preferred embodiments. It is advantageous to use a stable but also flexible substrate so that the electrodes are thus protected against excessive forces, as can occur during respective methods, manufacturing or the "activation" of the absorbent articles. Printing directly into a backsheet of the absorbent article would interfere with a capacitance measurement, as the electrodes would be in direct contact with the absorber material. Ink printed on a backsheet of the absorbent article can become unstable due to contraction, delamination and tear-off of the printed electrodes even when subjected to small forces, and much thinner layers of electrodes would be required. This would result in a resistance of the electrodes of well over 100 kOhm, which in turn makes capacitance measurement more difficult and greatly increases the measurement noise. Also, conductivity inhomogeneities can be reduced. The properties of the moisture sensor strip are also easier to adjust during production than when printing directly onto the backsheet.

In a further preferred embodiment, the two interdigitated electrodes are electrically insulated from each other and comprise electrode fingers having a length of less than 100 mm, preferably less than 50 mm and further preferably less than 10 mm and a distance from each other of 1 mm to 5 mm, preferably 1 mm to 4 mm, further preferably 1 mm to 3 mm and particularly preferably of 2 mm. The sensing depth is approximately the distance between the individual fingers, which limits the sensing to the absorbing material without including parts of the body in the measurement. Also, the finger structure increases the sensing area without increasing the sensing depth compared to just using two parallel conductive lines. Wide parallel lines are not robust against external interferences because of its high sensing depth. Therefore, the dimensions according to the invention are particularly suitable for a precisely defined measurement with a delimited measuring range.

In a further preferred embodiment, the two interdigitated electrodes are designed to generate a field with a penetration depth of 1 mm to 10 mm, preferably 1.3 mm to 4 mm, further preferably less than 1.6 mm to 3 mm and particularly preferably 2 mm to 2.5 mm. This achieves a particularly advantageous balance between penetration depth and susceptibility to external interference, as the sensor works bidirectionally in principle. A penetration depth that is too low only measures the surface, whereas a penetration depth that is too high also measures into the person's body and allows more external interference. Through this, the design of the IDE is optimized so that the penetration depth of the generated electric field does not exceed 10 mm during impedance measurements, which means that any change in the capacitance value can be attributed to the presence of excrement (urine, stool) in the absorbent article and not to the body of the user/patient. The pattern of the IDE preferably repeats in one direction, which means that the moisture sensor strip is suitable for being cut according to the size of the absorbent article to which it is applied. The IDE can be contacted at any point along the electrode perimeter/edge. The offset capacitance (initial capacitance) of the sensor is determined by the sensor length, the type of absorbent article, and the attachment method, such as type or thickness of adhesive. These specifications are predefined for a specific type of absorbent article.

According to a further preferred embodiment of the invention, the two interdigitated electrodes each and/or each electrode finger have a width of 1 mm to 4 mm, preferably 1.4 mm to 3 mm, further preferably 1.8 mm to 2.5 mm and particularly preferably 2 mm. These dimensions make it possible to reduce the area of the sensor to such an extent that the measurement noise remains manageable and the signal is so reliable that an evaluation algorithm can work with it.

In a further preferred embodiment, the two interdigitated electrodes have an ohmic resistance of less than 25 kOhm per 55 cm, preferably less than 20 kOhm per 55 cm, further preferably less than 15 kOhm per 55 cm and particularly preferably less than 14 kOhm per 55 cm. 14 kOhm per 55 cm is the maximum value up to which a particularly reliable capacitance measurement is possible. In addition, there is an increased mixture of the real and imaginary parts of the impedance, which means that the resistance has an influence on the capacitance. Furthermore, the greater the electrode resistance, the greater the measurement noise. Particularly preferred, all of the values mentioned here are per each electrode of the two interdigitated electrodes.

According to a further preferred embodiment, a deviation of the ohmic resistance is at most 29 %, preferably at most 27 % and particularly preferably at most 25 %. This ensures that the ohmic resistance and its real share of the total resistance do not exceed a certain maximum and do not affect the capacitive measurement, so that reliable measured values can be obtained.

In a further preferred embodiment, the polymer substrate has a height of 10 µm to 500 µm, preferably 35 µm to 65 µm, further preferably 45 µm to 55 µm and particularly preferably of 50 µm. 50 um thickness used for the height, results in a not too thin substrate that it still is easy to handle and cure, and not too thick that the substrate becomes stiff, uncomfortable or more expensive. Instead, the substrate remains flexible. The extensibility is reduced, which leads to a higher stability of the ink. Usually, absorbent articles are activated before use, a force is applied parallel to the absorbent article to loosen absorber material. If the ink is not printed on a stable substrate, the stretching can damage the electrodes. In this respect, such a continuous reel is particularly suitable for use in the corresponding process, as the risk of damage is reduced by the selected dimensions. The use of adhesives such as hot-melt adhesives in particular also ensures that the polymer substrate, in particular the PET substrate, retains its structure and functionality due to the effect of heat when the moisture sensor strip is bonded to the absorbent article and/or the cover layer.

The polymer, in particular PET, substrate preferably has a thermal conductivity of less than 1.0 W/(m*K), preferably less than 0.5 W/(m*K) and particularly preferably less than 0.2 W/(m*K). This prevents an excessive amount of heat from reaching the ink electrodes and damaging them.

In contrast, the ink preferably has a thermal conductivity of more than 100 W/(m*K), preferably more than 125 W/(m*K) and particularly preferably more than 150 W/(m*K).

In a preferred embodiment of the present disclosure, the electrodes have a height of 1 µm to 50 µm, preferably 1 µm to 16 µm, further preferably 8 µm to 14 µm, further preferably 10 µm to 12 µm, and particularly preferably of 10 µm and are arranged coplanar on the polymer substrate. These ranges have proven to be particularly stable and resistant in the corresponding methods. With a thinner layer, mechanical abrasion can damage the electrode material. Furthermore, the resistance becomes very high with layers that are too thin. Layers that are too thick can impair the flexibility of the material. Furthermore, a cost-benefit optimum is also achieved, taking the above criteria into account. In particular when using adhesives such as hot melt adhesives, it is also achieved that the ink retains its structure and functionality during bonding of the moisture sensor strip to the absorbent article and/or the cover layer through the effect of heat or mechanical action.

Preferably, the moisture sensor strip has a length of 400 mm to 750 mm, preferably 450 mm to 700 mm, further preferably 500 mm to 650 mm, further preferably 520 mm to 600 mm and particularly preferably 546 mm. In other words, the cut-to-length electrodes and the moisture sensor strip have these respective dimensions.

According to another preferred embodiment of the invention, at least one of the first adhesive agent, the second adhesive agent, and the third adhesive agent is a one of a construction adhesive, a cold melt adhesive, and a hot melt adhesive. A construction adhesive can be easily applied on an absorbent article in a manufacturing line at high speeds. Cold melt, which could be described as a hot melt adhesive with a lower melting point and application temperature, typically around 130 °C or less, and holt melt could also be used. This is applied with heated applicator on manufacturing line and allows very high speeds. Due to the homogeneous thickness, application with slot nozzles is preferable to spray application or segmented application.

In a preferred embodiment of the present disclosure, the hot melt adhesive has a processing temperature of 115° C to 140° C. In other words, the hot melt adhesive has a service or melting temperature of 115° C to 140° C. This means that the hot melt adhesive is used at 115 to 140°C, which is outside the normally permissible temperature range of PET, for example, which is a maximum of 110°C. Surprisingly, however, it has been shown that there is no impairment of the substrate, the cover layer or the printed ink. This therefore applies to each of the methods according to the invention for which the continuous reel is suitable. This thus relates to methods for producing the continuous reel (basic embodiment, preferred embodiments of the basic embodiment and first embodiment of the continuous reel according to the invention) and to use within the scope of the method according to the second preferred embodiment of the invention or a variant thereof. This is particularly effective in conjunction with the first and third adhesive, but also in conjunction with the second adhesive. The use of the hot melt adhesive, particularly in conjunction with the aforementioned temperature ranges, enables a particularly uniform application of adhesive. This in turn has an advantageous effect on measuring processes and the measurements obtained, as no local differences in the thickness of the adhesive layer and thus conductivity inhomogeneities can occur or be reduced. The distance between the corresponding layers formed by the cover layer, moisture sensor strips and electrodes and between them and the absorbent article is also particularly uniform. Reliable adhesion is also achieved. Compared to pressure-sensitive adhesives, exerting too much pressure on the system is avoided.

According to another preferred embodiment of the invention, the hot melt adhesive has a height of less than 10 µm, preferably less than 7 µm and particularly preferably less than 5 µm. This further enhances the aforementioned effects and further optimizes the interaction of adhesion, uniformity and adhesion of the adhesive layer, reliability of the electrodes, prevention of damage to the cover layer, moisture sensor strip or electrodes and distances to each other.

In a further preferred embodiment, the substrate has an elastic limit (yield strength) that is between 1.5 and 2.5 % strain, preferably at 2% strain, of the substrate. In other words, an elastic limit of the substrate is a value of mechanical stress (N/m²) below which the material is elastic, which occurs at a relative elongation between 1.5 and 2.5 %, preferably at 2%. The yield point is the point on a stress-strain curve that indicates the limit of elastic behavior and the beginning of plastic behavior. Below the yield point, a material will deform elastically and will return to its original shape when the applied stress is removed. Once the yield point is passed, some fraction of the deformation will be permanent and non-reversible and is known as plastic deformation. Yield strength testing (ISO 6892-1) involves taking a small sample with a fixed cross-section area and then pulling it with a controlled, gradually increasing force until the sample changes shape or breaks. This is called a tensile test. Longitudinal and/or transverse strain is recorded using mechanical or optical extensometers. It has been shown that the functionality of the electrodes (or compliance with the specifications) is guaranteed as long as the elongation of the substrate remains within this yield point or elastic limit.

Further preferred, at least one of the first adhesive agent, the second adhesive agent, and the third adhesive agent is a pressure-sensitive adhesive, preferably comprising acrylate polymer, rubber, natural rubber and/or synthetic thermoplastic elastomer. Pressure-sensitive polyacrylate (PSA) based adhesives can be screen printed onto the absorbent article, moisture sensor strip and/or cover layer and cured. This then allows subsequent adhering of the corresponding components. Alternatively, pressure-sensitive adhesives in the form of precured transfer tape can be used. PSA are useful for home care / care homes, as the continuous reel can be preferably delivered with adhesive on release paper/liner for easy attachment.

In a further preferred embodiment, the first adhesive is an electrically insulating adhesive film with a thickness of 1 to 100 µm, preferably 5 to 75 µm and further preferably 10 to 50 µm. The first adhesive provides additional insulation against possible liquids while at the same time ensuring a reliable bond between the moisture sensor strip and the absorbent article.

According to another preferred embodiment of the invention, the pressure-sensitive adhesive is printed to the absorbent article and/or the moisture sensor strip, wherein the pressure-sensitive adhesive is cured. Preferably the pressure-sensitive adhesive is screen-printed. Pressure-sensitive adhesives thus decouple preparation and provision of an adhesive and the actual adhering process.

The cover layer preferably comprises a first layer comprising the non-woven material, the non-woven material preferably comprising polypropylene and/or PET.

Further preferred, the cover layer comprises a first layer and a second layer, wherein the first layer comprises the non-woven material, the non-woven material preferably comprising polypropylene and/or PET, and wherein the second layer comprises a polymer film, preferably a polyethylene film. Through the selection of materials, the cover layer has sufficient tensile strength to allow processing in the absorbent article production line and to allow the formation of a stable pocket that will not break, but is pliable enough for a potential clip to pierce the material.

A further aspect of the present disclosure relates to a use of the first continuous reel in the method according to the second preferred embodiment of the invention or a variant thereof.

Another aspect of the present disclosure relates to an apparatus for use in the method according to the third preferred embodiment (retrofitting) of the invention, comprising a first roll for receiving a continuous reel according to the basic embodiment of the continuous reel according to the invention or a preferred embodiment thereof and a second roll for receiving the removed release paper. Further the apparatus comprises a first pressure roller and a second pressure roller, a gap formed with variable width between the first pressure roller and the second pressure roller and a coupling element configured for operatively connecting the first roller and the second roller and for synchronizing a rotation of the first roller and the second roller. Moreover, the apparatus comprises a preloaded spring disposed on the first pressure roller or the second pressure roller and configured for adjusting the pressure of the pressure rollers by adjusting the width of the gap. This apparatus makes it possible to subsequently connect already manufactured absorbent articles to a segment of the continuous reel and thereby attach a sensor for moisture measurement. The absorbent article is pushed into the gap. There, a portion of a moisture sensor strip and/or a portion of a cover layer is brought into contact with the absorbent article. The release paper has been removed beforehand so that a first adhesive agent and/or a second adhesive agent contact the absorbent article. Furthermore, if necessary because parts of the segment could still be covered by the release paper, the release paper is continuously removed during the insertion. Adhering takes place as a result of the pressure exerted by the pressure rollers. During this process, the absorbent article is progressively inserted so that a complete one of the plurality of segments is gradually bonded to the absorbent article. The absorbent article is then removed. The applied segment can be separated before or after removal. The two rolls are connected by the coupling element to ensure permanent tension on the release paper and not to interrupt the unwinding process. Herein, there is provided a device which makes it possible in a simple and quick manner to connect an absorbent article to a continuous reel according to the invention and thus to subsequently convert it into an absorbent article capable of performing a moisture measurement.

Another aspect of the present disclosure relates to a system manufactured in the basic embodiment of the method according to the invention, comprising an absorbent article, a continuous strip comprising a moisture sensor strip with two interdigitated electrodes printed with conductive materials-based ink on a PET substrate, and a cover layer and an access opening. The system further comprises a first adhesive agent disposed between a first partial surface of the absorbent article and a second surface of the moisture sensor strip except for a pocket region of the absorbent article, a second adhesive agent disposed between a second partial surface of the absorbent article and a first surface of the cover layer, wherein in the pocket region of the absorbent article the second adhesive is disposed on lateral edge regions of the cover layer and a region between the lateral edge regions is free of the second adhesive agent and wherein the cover layer overlaps both longitudinal edges of the moisture sensor strip. Moreover, the system comprises a pocket space confined by the pocket region of the absorbent article, by the lateral edge regions of the cover layer and by the region between the lateral edge regions, the pocket space being accessible via the access opening. The system further includes a clip comprising an integrated circuit, a first pivotable element, a second pivotable element connected to the first pivotable element at a folding edge, and teeth, wherein the first pivotable element is disposed in the pocket space. Further, the cover layer is pierced by the teeth in a closed position of the clip for attaching the clip to the cover layer, and the clip is aligned with the moisture sensor strip such that contact pads of the moisture sensor strip and contact pins of the clip are conductively connected to each other and such that the moisture sensor strip is aligned between the teeth.

The system preferably further comprises a third adhesive agent at least intermittently disposed between a first surface of the moisture sensor strip and the first surface of the cover layer material.

The system comprises a clip comprising an integrated circuit, a first pivotable element, a second pivotable element connected to the first pivotable element at a folding edge, and teeth, wherein the first pivotable element disposed in the pocket space, wherein the cover layer is pierced by the teeth in a closed position of the clip for attaching the clip to the cover layer, and wherein the clip aligned with the moisture sensor strip such that contact pads of the moisture sensor strip and contact pins of the clip are conductively connected to each other and such that the moisture sensor strip is aligned between the teeth. By using an integrated circuit, a variable frequency voltage is applied to the IDE and the current response measured between the electrodes. A frequency dependent impedance and, based thereon, frequency dependent capacitance is calculated. The clip preferably comprises an elastomer element, particularly preferably a silicone element. The silicone elements are configured for holding the moisture sensor stripe in place when the clip is in a closed position, ensuring that the electrical contact is maintained and that sufficient pressure is applied to ensure low contact resistance.

The clip connected to the moisture sensor strip preferably includes an integrated circuit capable of generating sinusoidal voltages at different frequencies. These voltages are applied to one electrode (driving electrode) of the moisture sensor strip. Another electrode (sensor electrode) is used to measure the current through the sensor. For each frequency step, the frequency-dependent capacitance is calculated from the voltage and current amplitude and the phase shift between them. This is equivalent to measuring the complex impedance and calculating the capacitance from the imaginary part. The real part is not used (ohmic resistance). The frequency-dependent capacitance is matched to a power law, and a value can be calculated from the matching coefficients, which is called the capacitance. The power law matching is also used for noise suppression.

The different embodiments of the disclosure described herein may be advantageously combined unless the contrary is indicated herein.

Reference is now made to the following figures, in order to describe preferred embodiments of the invention in more detail.
- Figure 1a: illustrates a first continuous reel in side view for use in the method according to the second preferred embodiment of the invention or a variant thereof;
- Figure 1b: illustrates a cross-section of a section of the first continuous reel from figure 1a;
- Figure 1c: illustrates a top-view of a section of the first continuous reel from figure 1a;
- Figure 2: illustrates a snapshot of a method for joining an absorbent article and a continuous strip comprising a moisture sensor strip and a cover layer according to a second preferred embodiment or a preferred embodiment thereof;
- Figure 3a: illustrates a part of the continuous strip from figure 2;
- Figure 3b: illustrates the joining of the absorbent article with the part of the continuous strip;
- Figure 3c: illustrates the absorbent article joint with the part of the continuous strip from figure 2;
- Figure 3d: illustrates the absorbent article joint with the part of the continuous strip from figure 3b in a perspective view;
- Figure 4a: illustrates a system with a partial section view according to the invention manufactured in a method according to the invention;
- Figure 4b: illustrates the system from figure 4a with a clip placed outside a pocket space;
- Figure 5a: illustrates an apparatus according to the invention for use in the method according to the third preferred embodiment (retrofitting) of the invention in a perspective view;
- Figure 5b: illustrates a cross-section of the apparatus from Figure 5a;
- Figure 6a: illustrates a method of joining an absorbent article and a continuous strip comprising a moisture sensor strip and a cover layer according to a basic embodiment of the invention;
- Figure 6b: illustrates a method of joining an absorbent article and a continuous strip comprising a moisture sensor strip and a cover layer according to a first variant of a second preferred embodiment;
- Figure 6c: illustrates a method of joining an absorbent article and a continuous strip comprising a moisture sensor strip and a cover layer according to a second variant of the second preferred embodiment;
- Figure 6d: illustrates a method of joining an absorbent article and a continuous strip comprising a moisture sensor strip and a cover layer according to a third preferred embodiment.

Reference will now be made in detail to embodiments which are illustrated in the drawings. Effects and features of the exemplary embodiments will be described with reference to the accompanying drawings. Therein, like reference numerals denote like elements, and redundant descriptions are omitted. The present invention, however, may be embodied in various different forms, and should not be construed as being limited to only the illustrated embodiments herein. Rather, these embodiments are provided solely as examples for fully conveying the aspects and features of the present invention to those skilled in the art.

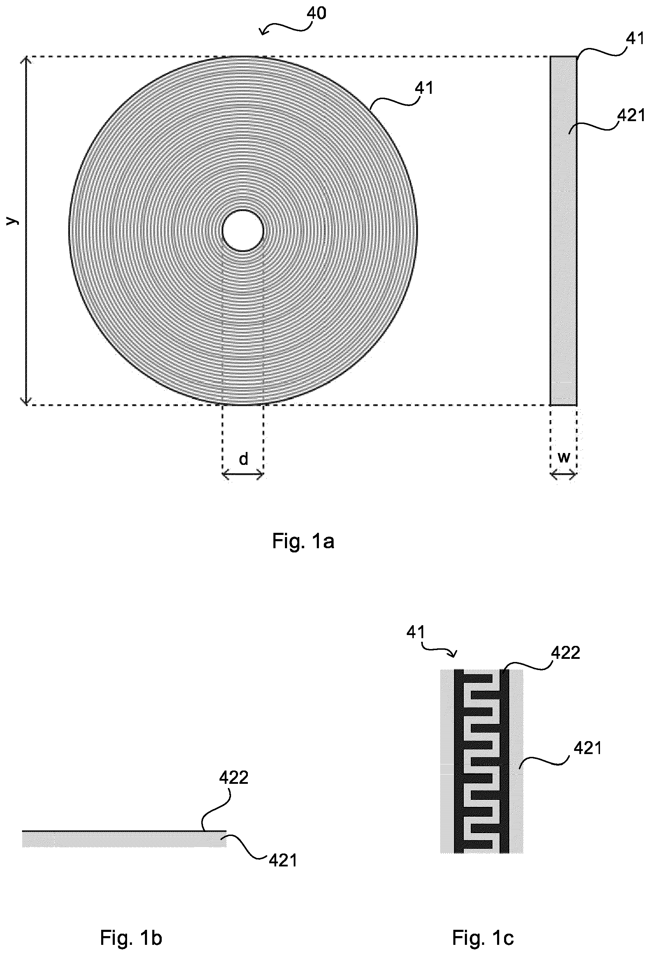

Figure 1a illustrates a first continuous reel 40 in side view for use in the method according to the second preferred embodiment of the invention or a variant thereof. The first continuous reel 40 comprises a moisture sensor strip 41 comprising two interdigitated electrodes, IDE, 422. Preferably, the IDE 422 are screen-printed with conductive carbon-based ink on a flexible and planar PET substrate 421 and cured. The moisture sensor strip 41 is provided on a roll formed by the first continuous reel 40. The moisture sensor strip 41 can be used as stripes which are cut to size or in a roll-to-roll process, the size depending on the length of the absorbent article. The moisture sensor strip 41 is preferably produced using roll-to-roll screen printing, while other methods, such as flexographic or (roto)gravure may also be used. The inner diameter d and the outer diameter y as well as the width w are dependent from the application. The width w is preferably optimized for comfort and cost reasons. Higher signal would be obtained with larger sensor but cost increases and comfort decreases.

Figure 1b shows a cross-section of a section of the first continuous reel. Here, the relationship between the height of the IDE 422 printed with conductive carbon-based ink and the thickness of the PET substrate 421 is qualitatively illustrated. The PET substrate 421 preferably has a thickness of 50 µm so that it is thin enough to be easily handled and cured, and not so thick that it becomes stiff, uncomfortable, or more expensive.

Figure 1c shows a top-view of a section of the first continuous reel 40 from figure 1a. It shows a preferred design of the IDE 422 disposed on the moisture sensor strip 41, which is printed on the PET substrate 421. The IDE 422 have preferably a height of approximately 12 µm in a co-planar layout. The electrode fingers of the IDE 422 have preferably a length of less than 10 mm and a spacing of 1 mm to 2 mm. The design of the IDE 422 is optimized in a way that a penetration depth of a generated electrical field during an impedance measurement is no greater than 5 mm, meaning that that any change in the capacitance value can be ascribed to the presence of excrement (urine, stool) inside an absorbent article, and not to body of the user/patient. The pattern of the IDE 422 repeats in one direction, which means that the first continuous roll 40 can be cut according to the size of the absorbent article to which it could be attached. The IDE 422 can be contacted at any point along the electrode perimeter/edge. The offset (starting) capacitance of a sensor will be determined by a length of an absorbent article, a type of an absorbent article, and means attachment (e.g. adhesive type or thickness). These specifications are pre-defined for a specific type of an absorbent article.

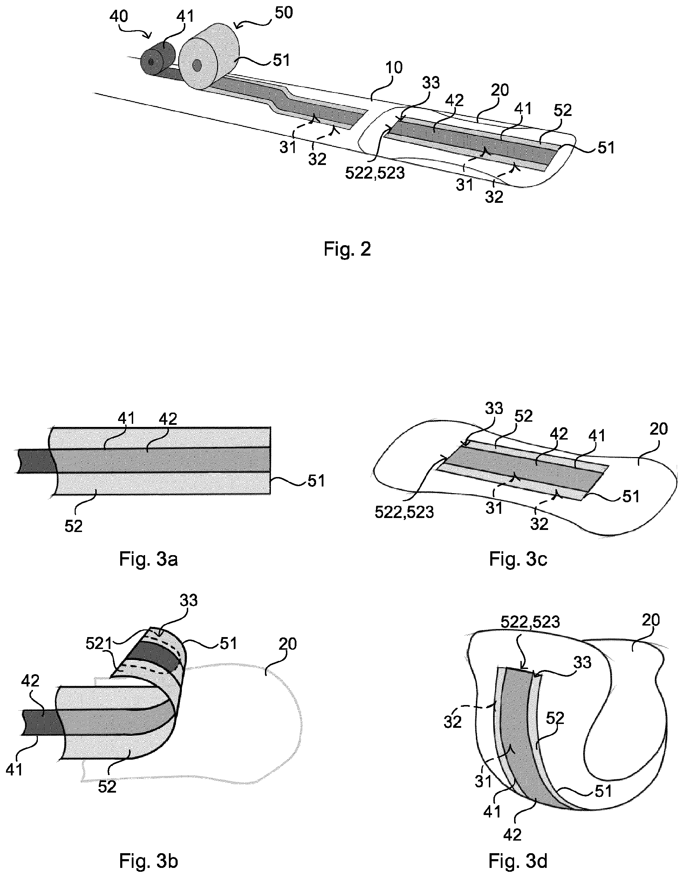

Figure 2 illustrates a snapshot of a method for joining an absorbent article 10 and a continuous strip comprising a moisture sensor strip 41 and a cover layer 51 according to a second preferred embodiment or a preferred embodiment thereof. Absorbent articles include, in particular, template products that are converted into a final product. The absorbent article 10, also called absorbent article pre-product 10, is such a template product. It is available as a quasi-endless raw material that can be processed in further process steps. It is assumed here that the finished absorbent articles 20, also called absorbent article finish-product 20, in this case diapers 20, are obtained from the quasi-endless absorbent article 10 by cutting.

The cover layer 51 is preferably breathable and comprises a non-woven material, such as polypropylene or PET.

As shown in the left area of Figure 2, the absorbent article 10 is located at the bottom, a first continuous reel 40 is located above it, and a second continuous reel 50 is located at the top. The first continuous reel 40 is preferably the one shown in figures 1a-1c. The first continuous reel 40 thus comprises a moisture sensor strip 41 and the second continuous reel 50 comprises a cover layer 51, wherein the moisture sensor strip 41 and the cover layer 51 are both unrolled from the reels 40, 50. The present illustrated snapshot of the roll-to-roll process comprises both the first variant of the second preferred embodiment and the second variant of the second preferred embodiment.

Not shown but present are also a first adhesive agent applied between the moisture sensor strip 41 and a first partial surface 31 for adhering a portion of the moisture sensor strip 42 to the first partial surface 31 of the absorbent article 10, 20, wherein in a pocket region 33 of the absorbent article 10, 20 there is no adhesion of the moisture sensor strip 41 to the first partial surface 31 and a second adhesive agent applied between the cover layer 51 and a second partial surface 32 for adhering a portion of the cover layer 52 to the second partial surface 32 of the absorbent article 10, 20, wherein in the pocket region 33 of the absorbent article 10, 20 lateral edge regions 521 of the cover layer 51 are adhered to the absorbent article 10, 20, while a region between the lateral edge regions 521 remains adhesive-free and wherein the cover layer 51 overlaps both longitudinal edges of the moisture sensor strip 41.

As shown in the right area of Figure 2, separating edges of the absorbent article finish-product 20 are already indicated. The portion of the moisture sensor strip 42 is placed on the absorbent article 20 and adhered to the first partial surface 31 of the absorbent article 20. In addition, the portion of the cover layer 52 is adhered to the second partial surface 32 of the absorbent article 20 and the cover layer 51 overlaps both longitudinal edges of the moisture sensor strip. In the pocket region 33 of the absorbent article 20 lateral edge regions 521 of the cover layer 51 are adhered to the absorbent article 20, while a region between the lateral edge regions 521 remains adhesive-free (see figure 3b for the lateral edge regions 521). In figure 2 an access opening 522 to a pocket space 523 is indicated confined by the pocket region 33 of the absorbent article 20, by the lateral edge regions 521 of the cover layer 51 and by the region between the lateral edge regions 521, the pocket space 523 being accessible via the access opening 522.

In case of the first variant or second variant of the second embodiment of the invention there is also present but not shown a third adhesive agent applied between at least a part of the moisture sensor strip 41 and a part of the cover layer 51.

Figure 3a illustrates a part of the continuous strip from figure 2, wherein this refers to the first variant of the second preferred embodiment, wherein before adhering the portion of the moisture sensor strip 42 to the first partial surface 31 of the absorbent article 10, 20 and before adhering the portion of the cover layer 52 to the second partial surface 32 of the absorbent article 10, 20, the moisture sensor strip 41 is adhered to the cover layer 51 by the third adhesive agent applied between at least a part of the moisture sensor strip 41 and a part of the cover layer 51. Therefore, a kind of semi-finished product or laminate is present, which is then subsequently adhered to the absorbent article 10, 20 as previously described.

Figure 3b illustrates the joining of the absorbent article with the part of the continuous strip from figure 3b. The dashed line shows the region between the two lateral edge regions 521 within which, on the one hand, the cover layer 51 is not adhered to the absorbent article 20 and, on the other hand, preferably the moisture sensor strip 41 is also not adhered to the absorbent article 20 and the cover layer 51. The dashed line at the same time shows the pocket region 33.

Figure 3c illustrates the absorbent article finish-product 20 joint with the part of the continuous strip from figure 2. The absorbent article finish-product 20 is separated from the absorbent article pre-product 10 along the separation edges and represents the final product.

Figure 3d illustrates the absorbent article joint with the part of the continuous strip from figure 3b in a perspective view. Here it is indicated that due to the materials used for both the cover layer 51 and the moisture sensor strip 41, the absorbent article finish-product 20 in the assembled form still retains its flexibility, while at the same time reliably holding the joint due to the appropriately selected adhesive agents.

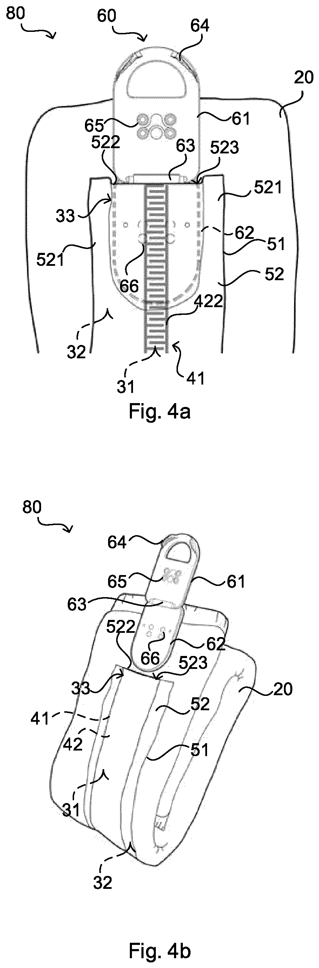

Figure 4a illustrates a system 80 with a partial section view according to the invention manufactured in a method according to the invention. The system 80 comprises the absorbent article finish-product 20, a continuous strip comprising a moisture sensor strip 41 with two interdigitated electrodes 422 printed with conductive materials-based ink on a PET substrate 421 and a cover layer 51 and an access opening 522. The system 80 further comprises a not shown first adhesive agent disposed between the first partial surface 31 of the absorbent article 20 and a second surface of the moisture sensor strip 41 except for a pocket region 33 of the absorbent article 20. The second surface of the moisture sensor strip 41 faces an outer surface of the absorbent article 20. A not shown second adhesive agent is disposed between the second partial surface 32 of the absorbent article 20 and a first surface of the cover layer 51, wherein in the pocket region 33 of the absorbent article 20 the second adhesive is disposed on lateral edge regions 521 of the cover layer 51. The first surface of the cover layer 51 faces the outer surface of the absorbent article 20. A region between the lateral edge regions 521 is free of the second adhesive agent and the cover layer 51 overlaps both longitudinal edges of the moisture sensor strip 41. Further a pocket space 523 is shown, which is confined by the pocket region 33 of the absorbent article 20, by the lateral edge regions 521 of the cover layer 51 and by the region between the lateral edge regions 521. The pocket space 523 is accessible via the access opening 522. Also shown is a clip 60 comprising a not shown integrated circuit, a first pivotable element 61, a second pivotable element 62 connected to the first pivotable element 61 at a folding edge 63, and teeth 64. The first pivotable element 61 is disposed in the pocket space 523, wherein the cover layer 51 is pierced by the teeth 64 in a (not shown) closed position of the clip 60 for attaching the clip 60 to the cover layer 51. The clip 60 is aligned with the moisture sensor strip 41 such that not shown contact pads of the moisture sensor strip 41 and contact pins 66 of the clip 60 are conductively connected to each other and such that the moisture sensor strip 41 is aligned between the teeth 64. The teeth 64 of the clip 60 puncture the cover layer 51 to hold it in position. The pocket space 523 is preferably made tight which enhances the alignment of the clip 60 to the moisture sensor strip 41. The dimensions of the pocket space 523 are configured in such a way that the contact pins align correctly and easily with the perimeter conducting tracks of the IDE 422, and at the same time the clip 60 is kept securely in place. When the clip 60 is not inserted, for example when the absorbent article 20 is packaged, no volume/bulk is added to the absorbent article 20. A non-woven with a sufficiently low grammage is chosen that allows the teeth to pierce the pocket space 523 further ensuring that the clip 60 is kept in place. The clip 60 squeezes the moisture sensor strip 41 in between the contact pins 66 of the clip 60 and the pivotable elements 61, 62 on the folding edge 63. This puts pressure on the backside of the moisture sensor strip 41 and presses it against the contact pins. Silicone pins 65 in the wearable clip hold the moisture sensor strip 41 in place when the clip is in its closed position, ensuring that the electrical contact is maintained and that sufficient pressure is applied to ensure low contact resistance

Figure 4b illustrates the system 80 from figure 4a with the clip 60 placed outside the pocket space 523.

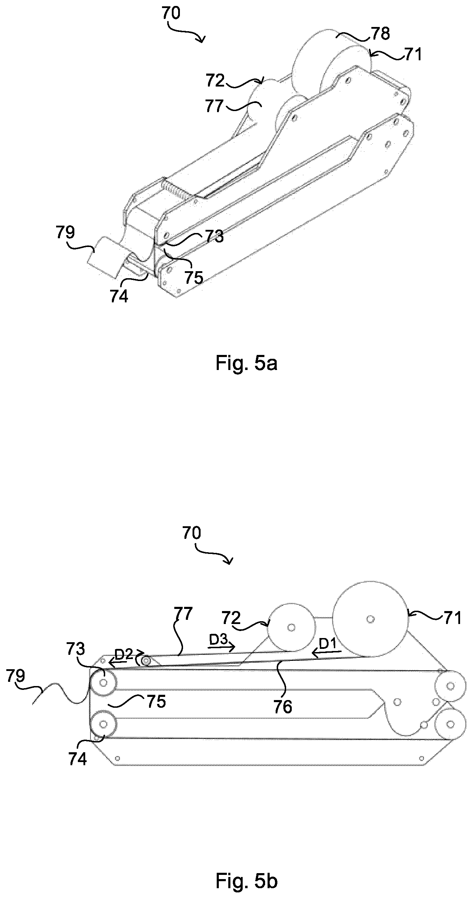

Figure 5a illustrates an apparatus 70 according to the invention for use in the method according to the third preferred embodiment (retrofitting) of the invention in a perspective view. The apparatus 70 comprises a first roll 71 for receiving a continuous reel 78 according to the first embodiment of a continuous reel according to the invention or a preferred embodiment thereof. The apparatus 70 further comprises a second roll 72 for receiving the removed release paper 77, a first pressure roller 73 and a second pressure roller 74. A gap 75 with variable width is formed between the first pressure roller 73 and the second pressure roller 74. A not shown coupling element is configured for operatively connecting the first roll 71 and the second roll 72 and for synchronizing a rotation of the first roll 71 and the second roll 72. An also not shown preloaded spring is disposed on the first pressure roller 73 or the second pressure roller 74 and configured for adjusting the pressure of the pressure rollers 73, 74 by adjusting the width of the gap 75. The continuous reel 78 disposed on the first roll 71 comprises a moisture sensor strip 41 comprising two interdigitated electrodes, IDE, 422 printed with conductive materials-based ink on a PET substrate, a cover layer 51 comprising a non-woven material and an access opening 522 to a pocket space 523 and a third adhesive agent at least intermittently disposed between a first surface of the moisture sensor strip 41 and a first surface of the cover layer 51 material, wherein no third adhesive agent is disposed in the pocket space 523. The continuous reel 78 further comprises a plurality of identical segments in a lengthwise direction of the continuous reel 78, wherein each segment comprises a first adhesive agent disposed on a second surface of the moisture sensor strip 41 except for a pocket portion of the moisture sensor strip 41, a second adhesive agent disposed on a first surface of the cover layer 51, wherein in a pocket portion of the cover layer 51 the second adhesive is disposed on lateral edge regions 521 of the pocket portion of the cover layer 51, wherein a region between the lateral edge regions 521 is free of the second adhesive agent, and at least one release paper covering the first adhesive agent and the second adhesive agent. The continuous reel 78 preferably comprises predefined separation areas, preferably perforations, for separating segments of the continuous reel 78, the separation areas being provided on the moisture sensor strip 41 and the cover layer 51. Also shown is an end 79 of a portion of a continuous strip 76, which is disposed in front of the first pressure roller 73 and the second pressure roller 74.

Figure 5b illustrates a cross-section of the apparatus from Figure 5a. Here, it is apparent that the portion of the continuous strip 76 comprising a portion of the moisture sensor strip 42 and a portion of the cover layer 52 is unrolled before being adhered to an absorbent article 10, 20. The direction of movement of the unrolled portion of the continuous strip 76 is represented by the moving direction D1. In addition, it is shown how removing the release paper takes place after unrolling the portion of the continuous strip 76 and before adhering the portion of the moisture sensor strip 42 and/or before adhering the portion of the cover layer 52 to the absorbent article 10, 20. The removed release paper 77 is deflected by a small roll, represented by the rotation arrow, and then after movement along the moving direction D3, the winding of the removed release paper 77 onto the release paper roll 72 takes place. The exposed portion of the moisture sensor strip 42 and/or portion of the cover layer 52 forms an end, and the absorbent article 10, 20 can receive the end 79 when the portion of the continuous strip 76 is inserted in the gap 7 by the absorbent article 10, 20.

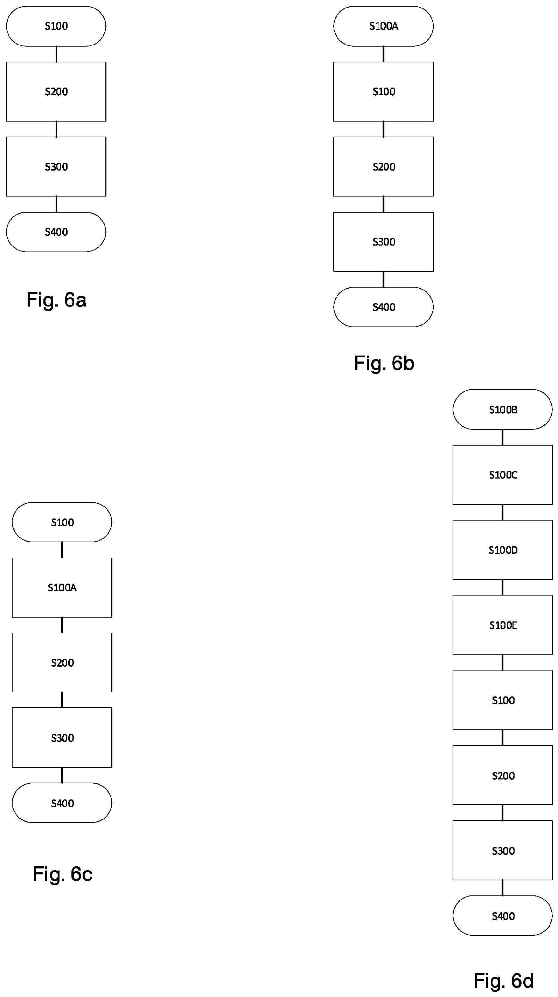

Figure 6a illustrates a method of joining an absorbent article and a continuous strip comprising a moisture sensor strip and a cover layer according to a basic embodiment of the invention. The method is explained with reference to figures 2 and figures 3a-3c, as this figure is particularly suitable for this form of roll-to-roll process, but is by no means limited to it. In a first step S100 a portion of the moisture sensor strip 42 is adhered to the first partial surface 31 of the absorbent article 10, 20 by applying the first adhesive agent between the moisture sensor strip 41 and the first partial surface 31, wherein in the pocket region 33 of the absorbent article 10, 20 there is no adhesion of the moisture sensor strip 41 to the first partial surface 31. The adhesion-free area is also shown in figure 3b by the dashed line. In a second step S200 the portion of the cover layer 52 is adhered to the second partial surface 32 of the absorbent article 10, 20 by applying the second adhesive agent between the cover layer 51 and the second partial surface 32, wherein in the pocket region 33 of the absorbent article 10, 20 lateral edge regions 521 of the cover layer 51 are adhered to the absorbent article 10, 20, while a region between the lateral edge regions 521 remains adhesive-free, which is also shown in figure 3b, and wherein the cover layer 51 overlaps both longitudinal edges of the moisture sensor strip (41). After step S200, in step S300 the adhered portion of the moisture sensor strip 42 and the adhered portion of the cover layer 52 are separated from the continuous strip. The steps S100 and S200 are very well represented by the left area of figure 2, whereas after step S300, i.e. separation, the situation is then as shown in the right area of figure 2. In the last step of the first embodiment the access opening 522 to the pocket space 523 confined by the pocket region 33 of the absorbent article 10, 20, by the lateral edge regions 521 of the cover layer 51 and by the region between the lateral edge regions 521, is provided, wherein the pocket space 523 is accessible via the access opening 522.

Figure 6b illustrates a method of joining an absorbent article and a continuous strip comprising a moisture sensor strip and a cover layer according to a first variant of a second preferred embodiment. Therefore, steps S100 to S400 do not differ in content. However, the process now starts with step S100A. In step S100A the moisture sensor strip 41 is adhered to the cover layer 51 by applying the third adhesive agent between at least a part of the moisture sensor strip 41 and a part of the cover layer 51. Therefore, Figure 2 no longer applies to this embodiment of the method according to the invention, since there the cover layer and the moisture sensor strip are at least not glued together before the moisture sensor strip is glued to the absorbent article. However, the other features such as relative arrangements etc. and the end product are still valid.

Figure 6c illustrates a method of joining an absorbent article and a continuous strip comprising a moisture sensor strip and a cover layer according to a second variant of the second preferred embodiment. The process shown there corresponds to the process shown in Figure 6b, except that the process starts with step S100, i.e. the adhering of the portion of the moisture sensor strip 41 to the first partial surface 31 of the absorbent article, followed by step S100A, i.e. the adhering of the moisture sensor strip 41 to the cover layer 51. Accordingly, the arrangement shown in the left area of figure 2 also applies to this embodiment of the method according to the invention.

Figure 6d illustrates a method of joining an absorbent article and a continuous strip comprising a moisture sensor strip and a cover layer according to a third preferred embodiment. The method is explained with reference to figures 4a and 4b as well as figures 5a and 5b, as this figure is particularly suitable for this form of retrofitting, but is by no means limited to it. Figures 5a and 5b show an apparatus for use in this described method. The continuous strip is provided as a continuous reel 78 comprising the moisture sensor strip 41 adhered to the cover layer 51 and wherein in step S100B a portion of the continuous strip 76 comprising the portion of the moisture sensor strip 42 and wherein the portion of the cover layer 52 is unrolled. In step S100C the release paper is removed. In step S100D the removed release paper which is deflected by the apparatus as shown in figure 5b is winded onto a release paper roll 72. The end of the portion of the continuous strip 76 is disposed in front of the first pressure roller 73 and the second pressure roller 74 and the absorbent article 10, 20 receives the end when the portion of the continuous strip 76 is inserted in the gap 75 by insertion of the absorbent article 10, 20. Afterwards in step S100E pressure is applied to the portion of the continuous strip with the pressure rollers 73, 74. Now follow the steps S100 to S400 already described, wherein adhering the portion of the moisture sensor strip 42 to the first partial surface 31 of the absorbent article 10, 20 and adhering a portion of the cover layer 52 to the second partial surface 32 of the absorbent article 10, 20 are presently performed simultaneously.

### Reference List

- 10: absorbent article pre-product
- 20: absorbent article finish-product
- 31: first partial surface
- 32: second partial surface
- 33: pocket region
- 40: first continuous reel
- 41: moisture sensor strip
- 42: portion of the moisture sensor strip
- 421: PET substrate
- 422: IDE
- 50: second continuous reel
- 51: cover layer
- 52: portion of the cover layer
- 521: lateral edge region
- 522: access opening
- 523: pocket space
- 60: clip
- 61: first pivotable element
- 62: second pivotable element
- 63: folding edge
- 64: teeth
- 65: silicon pins
- 66: contact pins
- 70: apparatus
- 71: first roll
- 72: second roll/release paper roll
- 73: first pressure roller
- 74: second pressure roller
- 75: gap
- 76: portion of the continuous strip
- 77: removed release paper
- 78: continuous reel
- 79: end of the portion of the continuous strip
- 80: system
- d: inner diameter
- y: outer diameter
- w: width of first continuous reel
- D1, D2, D3: moving directions
- S100A: adhering the moisture sensor strip to the cover layer
- S100B: unrolling the portion of the continuous strip
- S100C: removing the release paper
- S100D: winding the removed release paper onto a release paper roll
- S100E: applying pressure to the portion of the continuous strip
- S100: adhering a portion of the moisture sensor strip to a first partial surface of the absorbent article
- S200: adhering a portion of the cover layer to a second partial surface of the absorbent article
- S300: separating the adhered portion of the moisture sensor strip and the adhered portion of the cover layer from the continuous strip
- S400: providing an access opening to a pocket space

## Claims

1. A method of joining an absorbent article (10, 20) and a continuous strip comprising a moisture sensor strip (41) and a cover layer (51), the method comprising the steps of:
adhering a portion of the moisture sensor strip (42) to a first partial surface (31) of the absorbent article (10, 20) by applying a first adhesive agent between the moisture sensor strip (41) and the first partial surface (31), wherein in a pocket region (33) of the absorbent article (10, 20) there is no adhesion of the moisture sensor strip (41) to the first partial surface (31);
adhering a portion of the cover layer (52) to a second partial surface (32) of the absorbent article (10, 20) by applying a second adhesive agent between the cover layer (51) and the second partial surface (32), wherein in the pocket region (33) of the absorbent article (10, 20) lateral edge regions (521) of the cover layer (51) are adhered to the absorbent article (10, 20), while a region between the lateral edge regions (521) remains adhesive-free and wherein the cover layer (51) overlaps both longitudinal edges of the moisture sensor strip (41);
separating the adhered portion of the moisture sensor strip (42) and the adhered portion of the cover layer (52) from the continuous strip; and
providing an access opening (522) to a pocket space (523) confined by the pocket region (33) of the absorbent article (10, 20), by the lateral edge regions (521) of the cover layer (51) and by the region between the lateral edge regions (521), the pocket space (523) being accessible via the access opening (522),
wherein the pocket space (523) is configured for:
receiving a clip (60) comprising an integrated circuit, a first pivotable element (61) and a second pivotable element (62) connected to the first pivotable element (61) at a folding edge (63), wherein a size of the pocket space (523) is fitted to the size of the first pivotable element (61),
being pierced by teeth (64) of the second pivotable element (62) in a closed position of the clip (60) for attaching the clip (60) to the cover layer (51), and
aligning the clip (60) with the moisture sensor strip (41) such that contact pads of the moisture sensor strip (41) and contact pins (66) of the clip (60) are conductively connected to each other and such that the moisture sensor strip (41) is aligned between the teeth (64).

2. The method according to claim 1, wherein the continuous strip is provided as a continuous reel (78) comprising the moisture sensor strip (41) adhered to the cover layer (51) and wherein a portion of the continuous strip (76) comprising the portion of the moisture sensor strip (42) and the portion of the cover layer (52) is unrolled before being adhered to the absorbent article (10, 20).

3. The method according to claim 1, wherein the moisture sensor strip (41) is provided as a first continuous reel (40) and the cover layer (51) is provided as a second continuous reel (50).

4. The method of claim 3, wherein before adhering the portion of the moisture sensor strip (42) to the first partial surface (31) of the absorbent article (10, 20) and before adhering the portion of the cover layer (52) to the second partial surface (32) of the absorbent article (10, 20), the method further comprises:
adhering the moisture sensor strip (41) to the cover layer (51) by applying a third adhesive agent between at least a part of the moisture sensor strip (41) and a part of the cover layer (51).

5. The method according to claim 3, wherein after adhering the portion of the moisture sensor strip (42) to the first partial surface (31) of the absorbent article (10, 20), the method further comprises:
adhering the moisture sensor strip (41) to the cover layer (51) by applying a third adhesive agent between the moisture sensor strip (41) and the cover layer (51)

6. The method according to claim 5, wherein the moisture sensor strip (41) remains adhesive-free in the pocket region (33) of the absorbent article (10, 20) in the region between the lateral edge regions (521).

7. The method according to claim 2 or 4, wherein the moisture sensor strip (41) is intermittently adhered to the cover layer (51) and wherein a non-adhered region of the moisture sensor strip (41) is overlaid with the pocket region (33) before adhering the moisture sensor strip (41) to the absorbent article (10, 20).

8. Method according to claim 2, wherein the continuous reel (78) further comprises the first adhesive agent, the second adhesive agent and at least one release paper for covering the first adhesive agent and the second adhesive agent and wherein the method further comprises:
removing the release paper after unrolling the portion of the continuous strip (76) and before adhering the portion of the moisture sensor strip (42) and/or before adhering the portion of the cover layer (52) to the absorbent article (10, 20).

9. Method according to claim 8, wherein the method further comprises:
winding the removed release paper (77) onto a release paper roll (72); and
applying pressure to the portion of the continuous strip (76) with a pressure roller (73, 74) while adhering the portion of the moisture sensor strip (42) and the portion of the cover layer (52) to the absorbent article (10, 20),
wherein the continuous reel (78) and the release paper roll (72) are connected to each other by a transmission comprising a coupling.

10. Method according to claim 9, wherein the pressure of the pressure roller (73, 74) is adjusted by a preloaded spring.

11. Method according to claim 9 or 10, wherein the pressure is applied by a first pressure roller (73) and a second pressure roller (74) forming a gap (75) into which the absorbent article (10, 20) is inserted for adhering the portion of the moisture sensor strip (42) and the portion of the cover layer (52) to the absorbent article (10, 20).

12. Method according to claim 11, wherein an end of the portion of the continuous strip (76) is disposed in front of the first pressure roller (73) and the second pressure roller (74) and the absorbent article (10, 20) receives the end when the portion of the continuous strip (76) is inserted in the gap (75).

13. Continuous reel (78) of a continuous strip for use in the method according to any one of claims 1, 2 and 7 to 12, wherein the continuous reel (78) comprises:
a moisture sensor strip (41) comprising two interdigitated electrodes, IDE, (422) printed with conductive materials-based ink on a polymer substrate, prefereably polyethylenterephthalat, PET, substrate;
a cover layer (51) comprising a non-woven material and an access opening (522) to a pocket space (523);
a third adhesive agent at least intermittently disposed between a first surface of the moisture sensor strip (41) and a first surface of the cover layer (51) material, wherein no third adhesive agent is disposed in the pocket space (523); and
a plurality of identical segments in a lengthwise direction of the continuous reel (78), each segment comprising:
a first adhesive agent disposed on a second surface of the moisture sensor strip (41) except for a pocket portion of the moisture sensor strip (41);
a second adhesive agent disposed on a first surface of the cover layer (51), wherein in a pocket portion of the cover layer (51) the second adhesive is disposed on lateral edge regions (521) of the pocket portion of the cover layer (51) and a region between the lateral edge regions (521) is free of the second adhesive agent; and
at least one release paper covering the first adhesive agent and the second adhesive agent.

14. Apparatus (70) for use in method of claims 1 or 8 to 12, comprising
a first roll (71) for receiving a continuous reel (78) according to claim 13;
a second roll (72) for receiving removed release paper (77);
a first pressure roller (73) and a second pressure roller (74);
a gap (75) formed with variable width between the first pressure roller (73) and the second pressure roller (74);
a coupling element configured for operatively connecting the first roll (71) and the second roll (72) and for synchronizing a rotation of the first roll (71) and the second roll (72); and
a preloaded spring disposed on the first pressure roller (73) or the second pressure roller (74) and configured for adjusting the pressure of the pressure rollers (73, 74) by adjusting the width of the gap (75).

15. System (80) manufactured in the method according to claim 1, comprising
an absorbent article (10, 20);
a continuous strip comprising a moisture sensor strip (41) with two interdigitated electrodes (422) printed with conductive materials-based ink on a PET substrate (421), and a cover layer (51) and an access opening (522);
a first adhesive agent disposed between a first partial surface (31) of the absorbent article (10, 20) and a second surface of the moisture sensor strip (41) except for a pocket region (33) of the absorbent article (10, 20);
a second adhesive agent disposed between a second partial surface (32) of the absorbent article (10, 20) and a first surface of the cover layer (51), wherein in the pocket region (33) of the absorbent article (10, 20) the second adhesive is disposed on lateral edge regions (521) of the cover layer (51) and a region between the lateral edge regions (521) is free of the second adhesive agent and wherein the cover layer (51) overlaps both longitudinal edges of the moisture sensor strip (41);
a pocket space (523) confined by the pocket region (33) of the absorbent article (10, 20), by the lateral edge regions (521) of the cover layer (51) and by the region between the lateral edge regions (521), the pocket space (523) being accessible via the access opening (522); and
a clip (60) comprising an integrated circuit, a first pivotable element (61), a second pivotable element (62) connected to the first pivotable element (61) at a folding edge (63), and teeth (64),
wherein the first pivotable element (61) is disposed in the pocket space (523),
wherein the cover layer (51) is pierced by the teeth (64) in a closed position of the clip (60) for attaching the clip (60) to the cover layer (51), and
wherein the clip (60) is aligned with the moisture sensor strip (41) such that contact pads of the moisture sensor strip (41) and contact pins (66) of the clip (60) are conductively connected to each other and such that the moisture sensor strip (41) is aligned between the teeth (64).

## Patentansprüche

1. Verfahren zum Verbinden eines saugfähigen Artikels (10, 20) und eines kontinuierlichen Streifens, der einen Feuchtigkeitssensorstreifen (41) und eine Abdeckungsschicht (51) umfasst, wobei das Verfahren die Folgenden Schritte umfasst:
Kleben eines Abschnitts des Feuchtigkeitssensorstreifens (42) an eine erste Teilfläche (31) des saugfähigen Artikels (10, 20) durch Auftragen eines ersten Klebstoffs zwischen den Feuchtigkeitssensorstreifen (41) und die erste Teilfläche (31), wobei in einem Taschenbereich (33) des saugfähigen Artikels (10, 20) keine Haftung des Feuchtigkeitssensorstreifens (41) an der ersten Teilfläche (31) vorliegt;
Kleben eines Abschnitts der Abdeckungsschicht (52) an eine zweite Teilfläche (32) des saugfähigen Artikels (10, 20) durch Auftragen eines zweiten Klebstoffs zwischen die Abdeckungsschicht (51) und die zweite Teilfläche (32), wobei im Taschenbereich (33) des saugfähigen Artikels (10, 20) Seitenkantenbereiche (521) der Abdeckungsschicht (51) an dem saugfähigen Artikel (10, 20) kleben, während ein Bereich zwischen den Seitenkantenbereichen (521) klebstofffrei bleibt und wobei die Abdeckungsschicht (51) beide Längskanten des Feuchtigkeitssensorstreifens (41) überlappt;
Trennen des klebenden Abschnitts des Feuchtigkeitssensorstreifens (42) und des klebenden Abschnitts der Abdeckungsschicht (52) vom kontinuierlichen Streifen; und
Bereitstellen einer Zugangsöffnung (522) zu einem Taschenraum (523), der durch den Taschenbereich (33) des saugfähigen Artikels (10, 20), durch die Seitenkantenbereiche (521) der Abdeckungsschicht (51) und durch den Bereich zwischen den Seitenkantenbereichen (521) begrenzt ist, wobei der Taschenraum (523) über die Zugangsöffnung (522) zugänglich ist,
wobei der Taschenraum (523) konfiguriert ist, um:
eine Klammer (60) aufzunehmen, die eine integrierte Schaltung, ein erstes schwenkbares Element (61) und ein zweites schwenkbares Element (62) umfasst, das mit dem ersten schwenkbaren Element (61) an einer Faltkante (63) verbunden ist, wobei eine Größe des Taschenraums (523) an die Größe des ersten schwenkbaren Elements (61) angepasst ist,
durch Zähne (64) des zweiten schwenkbaren Elements (62) in einer geschlossenen Position der Klammer (60) durchdrungen zu werden, um die Klammer (60) an der Abdeckungsschicht (51) zu befestigen, und
die Klammer (60) an dem Feuchtigkeitssensorstreifen (41) so auszurichten, dass Kontaktflächen des Feuchtigkeitssensorstreifens (41) und Kontaktstifte (66) der Klammer (60) leitend miteinander verbunden sind und dass der Feuchtigkeitssensorstreifen (41) zwischen den Zähnen (64) ausgerichtet ist.

2. Verfahren nach Anspruch 1, wobei der kontinuierliche Streifen als kontinuierliche Rolle (78) bereitgestellt ist, die den Feuchtigkeitssensorstreifen (41) umfasst, der an der Abdeckungsschicht (51) klebt, und wobei ein Abschnitt des kontinuierlichen Streifens (76), der den Abschnitt des Feuchtigkeitssensorstreifens (42) und den Abschnitt der Abdeckungsschicht (52) umfasst, abgerollt wird, bevor er an den saugfähigen Artikel (10, 20) geklebt wird.

3. Verfahren nach Anspruch 1, wobei der Feuchtigkeitssensorstreifen (41) als eine erste kontinuierliche Rolle (40) bereitgestellt ist und die Abdeckungsschicht (51) als eine zweite kontinuierliche Rolle (50) bereitgestellt ist.

4. Verfahren nach Anspruch 3, wobei das Verfahren vor dem Kleben des Abschnitts des Feuchtigkeitssensorstreifens (42) an die erste Teilfläche (31) des saugfähigen Artikels (10, 20) und vor dem Kleben des Abschnitts der Abdeckungsschicht (52) an die zweite Teilfläche (32) des saugfähigen Artikels (10, 20) ferner Folgendes umfasst:
Kleben des Feuchtigkeitssensorstreifens (41) an die Abdeckungsschicht (51) durch Auftragen eines dritten Klebstoffs zwischen mindestens einen Teil des Feuchtigkeitssensorstreifens (41) und einen Teil der Abdeckungsschicht (51).

5. Verfahren nach Anspruch 3, wobei das Verfahren nach dem Kleben des Abschnitts des Feuchtigkeitssensorstreifens (42) an die erste Teilfläche (31) des saugfähigen Artikels (10, 20) ferner Folgendes umfasst:
Kleben des Feuchtigkeitssensorstreifens (41) an die Abdeckungsschicht (51) durch Auftragen eines dritten Klebstoffs zwischen den Feuchtigkeitssensorstreifen (41) und die Abdeckungsschicht (51)

6. Verfahren nach Anspruch 5, wobei der Feuchtigkeitssensorstreifen (41) im Taschenbereich (33) des saugfähigen Artikels (10, 20) im Bereich zwischen den Seitenkantenbereichen (521) klebstofffrei bleibt.

7. Verfahren nach Anspruch 2 oder 4, wobei der Feuchtigkeitssensorstreifen (41) intermittierend an der Abdeckungsschicht (51) klebt und wobei ein nicht klebender Bereich des Feuchtigkeitssensorstreifens (41) mit dem Taschenbereich (33) überlagert ist, bevor der Feuchtigkeitssensorstreifen (41) an den saugfähigen Artikel (10, 20) geklebt wird.

8. Verfahren nach Anspruch 2, wobei die kontinuierliche Rolle (78) ferner den ersten Klebstoff, den zweiten Klebstoff und mindestens ein Trennpapier zum Abdecken des ersten Klebstoffs und des zweiten Klebstoffs umfasst, und wobei das Verfahren ferner Folgendesumfasst:
Entfernen des Trennpapiers nach dem Abrollen des Abschnitts des kontinuierlichen Streifens (76) und vor dem Kleben des Abschnitts des Feuchtigkeitssensorstreifens (42) und/oder vor dem Kleben des Abschnitts der Abdeckungsschicht (52) an den saugfähigen Artikel (10, 20).

9. Verfahren nach Anspruch 8, wobei das Verfahren ferner Folgendes umfasst:
Wickeln des entfernten Trennpapiers (77) auf eine Trennpapierrolle (72); und
Ausüben von Druck auf den Abschnitt des kontinuierlichen Streifens (76) mit einer Druckrolle (73, 74), während der Abschnitt des Feuchtigkeitssensorstreifens (42) und der Abschnitt der Abdeckungsschicht (52) an den saugfähigen Artikel (10, 20) geklebt werden,
wobei die kontinuierliche Rolle (78) und die Trennpapierrolle (72) durch ein Getriebe, das eine Kupplung umfasst, miteinander verbunden sind.

10. Verfahren nach Anspruch 9, wobei der Druck der Druckrolle (73, 74) durch eine vorgespannte Feder angepasst wird.

11. Verfahren nach Anspruch 9 oder 10, wobei der Druck durch eine erste Druckrolle (73) und eine zweite Druckrolle (74) ausgeübt wird, die einen Spalt (75) bilden, in den der saugfähige Artikel (10, 20) eingesetzt wird, um den Abschnitt des Feuchtigkeitssensorstreifens (42) und den Abschnitt der Abdeckungsschicht (52) an den saugfähigen Artikel (10, 20) zu kleben.

12. Verfahren gemäß Anspruch 11, wobei ein Ende des Abschnitts des kontinuierlichen Streifens (76) vor der ersten Druckrolle (73) und der zweiten Druckrolle (74) angeordnet ist und der saugfähige Artikel (10, 20) das Ende aufnimmt, wenn der Abschnitt des kontinuierlichen Streifens (76) in den Spalt (75) eingesetzt wird.

13. Kontinuierliche Rolle (78) eines kontinuierlichen Streifens zur Verwendung in dem Verfahren nach einem der Ansprüche 1, 2 und 7 bis 12, wobei die kontinuierliche Rolle (78) Folgendes umfasst:
einen Feuchtigkeitssensorstreifen (41), umfassend zwei ineinandergreifende Elektroden, IDE, (422), die mit Tinte auf der Basis von leitenden Materialien auf ein Polymersubstrat, vorzugsweise Polyethylenterephthalat-, PET, -Substrat, gedruckt sind;
eine Abdeckungsschicht (51), umfassend ein Vliesmaterial und eine Zugangsöffnung (522) zu einem Taschenraum (523);
einen dritten Klebstoff, der zumindest intermittierend zwischen einer ersten Fläche des Feuchtigkeitssensorstreifens (41) und einer ersten Fläche des Materials der Abdeckungsschicht (51) angeordnet ist, wobei kein dritter Klebstoff im Taschenraum (523) angeordnet ist; und
eine Vielzahl von identischen Segmenten in einer Längsrichtung der kontinuierlichen Rolle (78), wobei jedes Segment Folgendes umfasst:
einen ersten Klebstoff, der auf einer zweiten Fläche des Feuchtigkeitssensorstreifens (41) angeordnet ist, mit Ausnahme eines Taschenabschnitts des Feuchtigkeitssensorstreifens (41);
einen zweiten Klebstoff, der auf einer ersten Fläche der Abdeckungsschicht (51) angeordnet ist, wobei in einem Taschenbereich der Abdeckungsschicht (51) der zweite Klebstoff auf Seitenkantenbereichen (521) des Taschenbereichs der Abdeckungsschicht (51) angeordnet ist und ein Bereich zwischen den Seitenkantenbereichen (521) frei von dem zweiten Klebstoff ist; und
mindestens ein Trennpapier, das den ersten Klebstoff und den zweiten Klebstoff abdeckt.

14. Vorrichtung (70) zur Verwendung in dem Verfahren nach den Ansprüchen 1 oder 8 bis 12, umfassend
eine erste Walze (71) zum Aufnehmen einer kontinuierlichen Rolle (78) nach Anspruch 13;
eine zweite Walze (72) zum Aufnehmen des entfernten Trennpapiers (77);
eine erste Druckrolle (73) und eine zweite Druckrolle (74);
einen Spalt (75), der mit variabler Breite zwischen der ersten Druckrolle (73) und der zweiten Druckrolle (74) ausgebildet ist;
ein Kopplungselement, das konfiguriert ist, um die erste Walze (71) und die zweite Walze (72) betriebsfähig zu verbinden und eine Drehung der ersten Walze (71) und der zweiten Walze (72) zu synchronisieren; und
eine vorgespannte Feder, die an der ersten Druckrolle (73) oder der zweiten Druckrolle (74) angeordnet ist und konfiguriert ist, um den Druck der Druckrollen (73, 74) durch Anpassen der Breite des Spalts (75) anzupassen.

15. System (80), das in dem Verfahren nach Anspruch 1 hergestellt wird, umfassend
einen saugfähigen Artikel (10, 20);
einen kontinuierlichen Streifen, umfassend einen Feuchtigkeitssensorstreifen (41) mit zwei ineinandergreifenden Elektroden (422), die mit Tinte auf der Basis von leitenden Materialien auf ein PET-Substrat (421) gedruckt sind, und eine Abdeckungsschicht (51) sowie eine Zugangsöffnung (522);
einen ersten Klebstoff, der zwischen einer ersten Teilfläche (31) des saugfähigen Artikels (10, 20) und einer zweiten Fläche des Feuchtigkeitssensorstreifens (41) mit Ausnahme eines Taschenbereichs (33) des saugfähigen Artikels (10, 20) angeordnet ist;
einen zweiten Klebstoff, der zwischen einer zweiten Teilfläche (32) des saugfähigen Artikels (10, 20) und einer ersten Fläche der Abdeckungsschicht (51) angeordnet ist, wobei im Taschenbereich (33) des saugfähigen Artikels (10, 20) der zweite Klebstoff auf Seitenkantenbereichen (521) der Abdeckungsschicht (51) angeordnet ist und ein Bereich zwischen den Seitenkantenbereichen (521) frei von dem zweiten Klebstoff ist und wobei die Abdeckungsschicht (51) beide Längskanten des Feuchtigkeitssensorstreifens (41) überlappt;
einen Taschenraum (523), der durch den Taschenbereich (33) des saugfähigen Artikels (10, 20), durch die Seitenkantenbereiche (521) der Abdeckungsschicht (51) und durch den Bereich zwischen den Seitenkantenbereichen (521) begrenzt ist, wobei der Taschenraum (523) über die Zugangsöffnung (522) zugänglich ist; und
eine Klammer (60), die eine integrierte Schaltung, ein erstes schwenkbares Element (61), ein zweites schwenkbares Element (62), das mit dem ersten schwenkbaren Element (61) an einer Faltkante (63) verbunden ist, und Zähne (64) umfasst,
wobei das erste schwenkbare Element (61) in dem Taschenraum (523) angeordnet ist,
wobei die Abdeckungsschicht (51) von den Zähnen (64) in einer geschlossenen Position der Klammer (60) durchdrungen wird, um die Klammer (60) an der Abdeckungsschicht (51) zu befestigen, und
wobei die Klammer (60) an dem Feuchtigkeitssensorstreifen (41) so ausgerichtet ist, dass Kontaktflächen des Feuchtigkeitssensorstreifens (41) und Kontaktstifte (66) der Klammer (60) leitend miteinander verbunden sind und dass der Feuchtigkeitssensorstreifen (41) zwischen den Zähnen (64) ausgerichtet ist.

## Revendications

1. Procédé d'assemblage d'un article absorbant (10, 20) et d'une bande continue comprenant une bande de capteur d'humidité (41) et une couche de recouvrement (51), le procédé comprenant les étapes suivantes :
coller une partie de la bande de capteur d'humidité (42) à une première surface partielle (31) de l'article absorbant (10, 20) en appliquant un premier agent adhésif entre la bande de capteur d'humidité (41) et la première surface partielle (31), dans lequel, dans une région de poche (33) de l'article absorbant (10, 20), il n'y a pas d'adhésion de la bande de capteur d'humidité (41) à la première surface partielle (31) ;
coller une partie de la couche de recouvrement (52) à une deuxième surface partielle (32) de l'article absorbant (10, 20) en appliquant un deuxième agent adhésif entre la couche de recouvrement (51) et la deuxième surface partielle (32), dans lequel, dans la région de poche (33) de l'article absorbant (10, 20), des régions de bord latéral (521) de la couche de recouvrement (51) sont collées à l'article absorbant (10, 20), tandis qu'une région entre les régions de bord latéral (521) reste sans colle et dans lequel la couche de recouvrement (51) chevauche les deux bords longitudinaux de la bande de capteur d'humidité (41) ;
séparer la partie collée de la bande de capteur d'humidité (42) et la partie collée de la couche de recouvrement (52) de la bande continue ; et
fournir une ouverture d'accès (522) à un espace de poche (523) confiné par la région de poche (33) de l'article absorbant (10, 20), par les régions de bord latéral (521) de la couche de recouvrement (51) et par la région entre les régions de bord latéral (521), l'espace de poche (523) étant accessible par l'ouverture d'accès (522),
dans lequel l'espace de poche (523) est configuré pour :
recevoir un clip (60) comprenant un circuit intégré, un premier élément pivotable (61) et un deuxième élément pivotable (62) relié au premier élément pivotable (61) au niveau d'un bord de pliage (63), dans lequel une taille de l'espace de poche (523) est adaptée à la taille du premier élément pivotable (61),
être percé par des dents (64) du deuxième élément pivotable (62) dans une position fermée du clip (60) pour fixer le clip (60) à la couche de recouvrement (51), et
aligner le clip (60) avec la bande de capteur d'humidité (41) de telle sorte que des plots de contact de la bande de capteur d'humidité (41) et des broches de contact (66) du clip (60) sont connectés de manière conductrice l'un à l'autre et de telle sorte que la bande de capteur d'humidité (41) est alignée entre les dents (64).

2. Procédé selon la revendication 1, dans lequel la bande continue est fournie sous forme d'une bobine continue (78) comprenant la bande de capteur d'humidité (41) collée à la couche de recouvrement (51) et dans lequel une partie de la bande continue (76) comprenant la partie de la bande de capteur d'humidité (42) et la partie de la couche de recouvrement (52) est déroulée avant d'être collée à l'article absorbant (10, 20).

3. Procédé selon la revendication 1, dans lequel la bande de capteur d'humidité (41) est fournie sous forme d'une première bobine continue (40) et la couche de recouvrement (51) est fournie sous forme d'une deuxième bobine continue (50).

4. Procédé selon la revendication 3, dans lequel, avant de coller la partie de la bande de capteur d'humidité (42) à la première surface partielle (31) de l'article absorbant (10, 20) et avant de coller la partie de la couche de recouvrement (52) à la deuxième surface partielle (32) de l'article absorbant (10, 20), le procédé comprend en outre :
coller la bande de capteur d'humidité (41) à la couche de recouvrement (51) en appliquant un troisième agent adhésif entre au moins une partie de la bande de capteur d'humidité (41) et une partie de la couche de recouvrement (51).

5. Procédé selon la revendication 3, dans lequel après avoir collé la partie de la bande de capteur d'humidité (42) à la première surface partielle (31) de l'article absorbant (10, 20), le procédé comprend en outre :
coller la bande de capteur d'humidité (41) à la couche de recouvrement (51) en appliquant un troisième agent adhésif entre la bande de capteur d'humidité (41) et la couche recouvrement (51).

6. Procédé selon la revendication 5, dans lequel la bande de capteur d'humidité (41) reste sans colle dans la région de poche (33) de l'article absorbant (10, 20) dans la région entre les régions de bord latéral (521).

7. Procédé selon la revendication 2 ou 4, dans lequel la bande de capteur d'humidité (41) est collée par intermittence à la couche de recouvrement (51) et dans lequel une région non collée de la bande de capteur d'humidité (41) est chevauchée à la région de poche (33) avant de coller la bande de capteur d'humidité (41) à l'article absorbant (10, 20).

8. Procédé selon la revendication 2, dans lequel la bobine continue (78) comprend en outre le premier agent adhésif, le deuxième agent adhésif et au moins un papier décollable pour couvrir le premier agent adhésif et le deuxième agent adhésif et dans lequel le procédé comprend en outre :
retirer le papier décollable après avoir déroulé la partie de la bande continue (76) et avant de coller la partie de la bande de capteur d'humidité (42) et/ou avant de coller la partie de la couche de recouvrement (52) à l'article absorbant (10, 20).

9. Procédé selon la revendication 8, dans lequel le procédé comprend en outre :
enrouler le papier décollable retiré (77) sur un rouleau de papier décollable (72) ; et
appliquer une pression sur la partie de la bande continue (76) à l'aide d'un rouleau presseur (73, 74) tout en collant la partie de la bande de capteur d'humidité (42) et la partie de la couche de recouvrement (52) à l'article absorbant (10, 20),
dans lequel la bobine continue (78) et le rouleau de papier décollable n (72) sont reliés l'un à l'autre par une transmission comprenant un couplage.

10. Procédé selon la revendication 9, dans lequel la pression du rouleau presseur (73, 74) est réglée par un ressort précontraint.

11. Procédé selon la revendication 9 ou 10, dans lequel la pression est appliquée par un premier rouleau presseur (73) et un deuxième rouleau presseur (74) formant un espace (75) dans lequel l'article absorbant (10, 20) est inséré pour coller la partie de la bande de capteur d'humidité (42) et la partie de la couche de recouvrement (52) à l'article absorbant.

12. Procédé selon la revendication 11, dans lequel une extrémité de la partie de la bande continue (76) est disposée devant le premier rouleau presseur (73) et le deuxième rouleau presseur (74) et l'article absorbant (10, 20) reçoit l'extrémité lorsque la partie de la bande continue (76) est insérée dans l'espace (75).

13. Bobine continue (78) d'une bande continue destiné à être utilisé dans le procédé selon l'une quelconque des revendications 1, 2 et 7 à 12, dans laquelle la bobine continue (78) comprend :
une bande de capteur d'humidité (41) comprenant deux électrodes interdigitées, IDE, (422) imprimées avec une encre à base de matériaux conducteurs sur un substrat polymère, de préférence un substrat en polyéthylentéréphtalate, PET ;
une couche de recouvrement (51) comprenant un matériau non tissé et une ouverture d'accès (522) à un espace de poche (523) ;
un troisième agent adhésif disposé au moins de manière intermittente entre une première surface de la bande de capteur d'humidité (41) et une première surface du matériau de couche de recouvrement (51), dans laquelle aucun troisième agent adhésif n'est disposé dans l'espace de poche (523) ; et
une pluralité de segments identiques dans un sens de longueur de la bobine continue (78), chaque segment comprenant :
un premier agent adhésif disposé sur une deuxième surface de la bande de capteur d'humidité (41), à l'exception d'une partie de la poche de la bande de capteur d'humidité (41) ;
un deuxième agent adhésif disposé sur une première surface de la couche de recouvrement (51), dans laquelle, dans une partie de la poche de la couche de recouvrement (51), le deuxième adhésif est disposé sur des régions de bord latéral (521) de la partie de poche de la couche de recouvrement (51) et une région entre les régions de bord latéral (521) est exempte du deuxième agent adhésif ; et
au moins un papier décollable recouvrant le premier agent adhésif et le deuxième agent adhésif.

14. Dispositif (70) destiné à être utilisé dans le procédé des revendications 1 ou 8 à 12, comprenant
un premier rouleau (71) destiné à recevoir une bobine continue (78) selon la revendication 13 ;
un deuxième rouleau (72) destiné à recevoir le papier décollable retiré (77) ;
un premier rouleau presseur (73) et un deuxième rouleau presseur (74) ;
un espace (75) formé avec une largeur variable entre le premier rouleau presseur (73) et le deuxième rouleau presseur (74) ;
un élément de couplage configuré pour connecter de manière opérationnelle le premier rouleau (71) et le deuxième rouleau (72) et pour synchroniser une rotation du premier rouleau (71) et du deuxième rouleau (72) ; et
un ressort précontraint disposé sur le premier rouleau presseur (73) ou le deuxième rouleau presseur (74) et configuré pour régler la pression des rouleaux presseurs (73, 74) en réglant la largeur de l'espace (75).

15. Système (80) fabriqué dans le procédé selon la revendication 1, comprenant
un article absorbant (10, 20) ;
une bande continue comprenant une bande de capteur d'humidité (41) avec deux électrodes interdigitées (422) imprimées avec une encre à base de matériaux conducteurs sur un substrat PET (421), ainsi qu'une couche de recouvrement (51) et une ouverture d'accès (522) ;
un premier agent adhésif disposé entre une première surface partielle (31) de l'article absorbant (10, 20) et une deuxième surface de la bande de capteur d'humidité (41), à l'exception d'une région de poche (33) de l'article absorbant (10, 20) ;
un deuxième agent adhésif disposé entre une deuxième surface partielle (32) de l'article absorbant (10, 20) et une première surface de la couche de recouvrement (51), dans lequel, dans la région de poche (33) de l'article absorbant (10, 20), le deuxième adhésif est disposé sur des régions de bord latéral (521) de la couche de recouvrement (51) et une région entre les régions de bord latéral (521) est exempte du deuxième agent adhésif et dans lequel la couche de recouvrement (51) recouvre les deux bords longitudinaux de la bande de capteur d'humidité (41) ;
un espace de poche (523) confiné par la région de poche (33) de l'article absorbant (10, 20), par les régions de bord latéral (521) de la couche de recouvrement (51) et par la région entre les régions de bord latéral (521), l'espace de poche (523) étant accessible par l'ouverture d'accès (522) ; et
un clip (60) comprenant un circuit intégré, un premier élément pivotable (61), un deuxième élément pivotable (62) relié au premier élément pivotable (61) au niveau d'un bord de pliage (63), et des dents (64),
dans lequel le premier élément pivotable (61) est disposé dans l'espace de poche (523),
dans lequel la couche de recouvrement (51) est percée par les dents (64) dans une position fermée du clip (60) pour fixer le clip (60) à la couche de recouvrement (51), et
dans lequel le clip (60) est aligné avec la bande de capteur d'humidité (41) de telle sorte que des plots de contact de la bande de capteur d'humidité (41) et des broches de contact (66) du clip (60) sont connectés de manière conductrice l'un à l'autre et de telle sorte que la bande de capteur d'humidité (41) est alignée entre les dents (64).
